(19) Europäisches Patentamt
European Patent Office
Office européen des brevets

(11) EP 2 486 007 B1

(12) # FASCICULE DE BREVET EUROPEEN

(45) Date de publication et mention de la délivrance du brevet:
**03.07.2013 Bulletin 2013/27**

(21) Numéro de dépôt: **10778695.6**

(22) Date de dépôt: **05.10.2010**

(51) Int Cl.:
***C07D 207/14*** (2006.01) ***C07D 207/16*** (2006.01)
***A61K 31/4015*** (2006.01) ***A61P 35/00*** (2006.01)

(86) Numéro de dépôt international:
**PCT/FR2010/052095**

(87) Numéro de publication internationale:
**WO 2011/042654 (14.04.2011 Gazette 2011/15)**

(54) **MODULATEURS PEPTIDIQUES ET PEPTIDOMIMETIQUES NON COMPETITIFS SPECIFIQUES DE LA GLYCOPROTEINE P**

SPEZIFISCHE NICHT KONKURRIERENDE PEPTID- UND PEPTIDOMIMETISCHE MODULATOREN DER P-GLYCOPROTEINE

SPECIFIC NON COMPETITIVE PEPTID AND PEPTIDOMIMETIC MODULATORS OF THE GLYCOPROTEIN P

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.10.2009 FR 0956954**

(43) Date de publication de la demande:
**15.08.2012 Bulletin 2012/33**

(73) Titulaires:
- **Centre National de la Recherche Scientifique CNRS**
  **75794 Paris Cedex 16 (FR)**
- **Université Claude Bernard Lyon 1**
  **69622 Villeurbanne Cedex (FR)**

(72) Inventeurs:
- **ARNAUD, Ophélie**
  **F-69003 Lyon (FR)**
- **KOUBEISSI, Ali**
  **F-51100 Reims (FR)**
- **ETTOUATI, Laurent**
  **F-69008 Lyon (FR)**
- **PARIS, Joëlle**
  **F-69003 Lyon (FR)**
- **DI PIETRO, Attilio**
  **F-01570 Manziat (FR)**
- **FALSON, Pierre**
  **F-07100 Annonay (FR)**

(74) Mandataire: **Novagraaf Technologies**
**122 rue Edouard Vaillant**
**92593 Levallois-Perret Cedex (FR)**

(56) Documents cités:
**US-A- 5 620 971**

- **MODOK S ET AL: "Modulation of multidrug resistance efflux pump activity to overcome chemoresistance in cancer", CURRENT OPINION IN PHARMACOLOGY, ELSEVIER SCIENCE PUBLISHERS, NL LNKD- DOI:10.1016/J.COPH.2006.01.009, vol. 6, no. 4, 1 août 2006 (2006-08-01), pages 350-354, XP025229676, ISSN: 1471-4892 [extrait le 2006-08-01]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 486 007 B1

## Description

### Domaine technique

La présente invention se rapporte à un composé de structure (I), se comportant comme un inhibiteur spécifique non compétitif de la glycoprotéine P (P-gp pour « Pleiotropic glycoprotein »), utile comme médicament, notamment pour l'amélioration de l'efficacité de traitements chimiothérapeutiques.

La présente invention trouve donc des applications notamment dans le domaine médical, notamment dans le cadre de traitements chimiothérapeutiques de cancers ou d'infections.

Dans la description ci-dessous, les références entre crochets ([]) renvoient à la liste des références présentée après les exemples.

### Etat de la technique

Les traitements chimiothérapeutiques sont actuellement utilisés pour lutter contre les cancers et les infections d'origine virale, bactérienne, fongique, ou parasitaire, en détruisant les cellules cibles du traitement que sont les cellules tumorales ou infectées par l'agent infectieux (virus, bactérie, champignon, parasite, etc.). Leur efficacité est souvent limitée par la résistance que développent les cellules cibles à l'encontre des agents chimiothérapeutiques administrés dans le cadre du traitement. Parmi les phénomènes de résistances acquises, la résistance à de multiples drogues médicamenteuses (« MultiDrug Resistance », également nommée MDR) se traduit par une diminution de la concentration intracellulaire de l'agent chimiothérapeutique. Les cellules cibles deviennent résistantes non seulement aux agents chimiothérapeutiques administrés, mais également à un grand nombre de molécules structuralement non apparentées.

Cette diminution de la concentration intracellulaire de l'agent chimiothérapeutique a pour origine l'expression massive de protéines de transport de type « ATP-Binding Cassette » (ABC), qui expulsent l'agent chimiothérapeutique hors des cellules cibles. Trois de ces protéines, appelées également pompes d'efflux, ont été identifiées chez l'homme :

- la glycoprotéine P (P-gp) (GOTTESMAN M. M., LING V. FEBS Lett., 2006, 580(4), 998-1009, [1]) encore appelée MDR1 ou ABCB1, découverte par Juliano et Ling en 1976 (Juliano, R. L. & Ling, V. A surface glycoprotein modulating drug permeability in Chinese hamster ovary cell mutants. Biochim Biophys Acta 455, 152-162, doi:0005-2736(76) 90160-7 [pii] 19976), [2])
- la MRP1 (Multidrug Resistance Protein 1), également appelée ABCC1, découverte par Cole et Deeley dans les cancers du poumon (Cole, S. P. et al. Overexpression of a transporter gene in a multidrug-resistant human lung cancer cell line. Science 258, 1650-1654 (1992), [3])
- la « Breast Cancer Resistance Protein » (BCRP) (DOYLE L. A., YANG W., ABRUZZO L. V., KROGMANN T., GAO Y., RISHI A. K., ROSS D. D. Proc. Natl. Acad. Sci. USA, 1998, 95(26), 15665-15670, [4]) encore appelée ABCG2 ou MXR (mitoxantrone resistance), découverte plus récemment par Ross *et al.* d'une part (Ross, D. D. et al. Atypical multidrug résistance: breast cancer resistance protein messenger RNA expression in mitoxantrone-selected cell lines. J Natl Cancer Inst 91, 429-433 (1999), [5]) et Bates et al. d'autre part (J. Cell Sci., 2000, 113(Pt 11), 2011-2021, [6]).

Ces protéines d'efflux sont incluses dans la membrane plasmique des cellules par un domaine transmembranaire lié à un large domaine intracellulaire composé de 2 deux sites de fixation de nucléotides (*Nucleotide-Binding Domain,* NBD) et d'un domaine extracellulaire. La structure tridimensionnelle de la P-gp a été récemment déterminée (Aller, S. G. et al. Structure of P-Glycoprotein Reveals a Molecular Basis for Poly-Specific Drug Binding. Science 323, 1718-1722, doi:10.1126/science.1168750 (2009), [7]), proche de celle des 2 protéines d'efflux procaryotiques, Sav1866 (Dawson, R. J. & Locher, K. P. Structure of a bacterial multidrug ABC transporter. Nature 443, 180-185 (2006), [8]) et MsbA (Ward, A., Reyes, C. L., Yu, J., Roth, C. B. & Chang, G. Flexibility in the ABC transporter MsbA: Alternating access with a twist. Proceedings of the National Academy of Sciences 104, 19005-19010, doi:10.1073/pnas.0709388104 (2007), [9]). Elles permettent l'efflux ATP-dépendant hors de la cellule d'agents cytotoxiques variés.

La P-gp (Ueda, K., Cardarelli, C., Gottesman, M. M. & Pastan, I. Expression of a Full-Length cDNA for the Human "MDR1" Gene Confers Resistance to Colchicine, Doxorubicin, and Vinblastine. Proceedings of the National Academy of Sciences 84, 3004-3008, doi:10.1073/pnas.84.9.3004 (1987), [10]) est la protéine la plus impliquée dans les phénomènes de résistance aux médicaments, renforcée dans son action par MRP1 ([3]) et BCRP1 (Litman, T. et al. The multidrug-resistant phenotype associated with overexpression of the new ABC half-transporter, MXR (ABCG2). J Cell Sci 113 (Pt 11), 2011-2021 (2000), [11]).

La P-gp a un rôle physiologique de protection de différents organes ; elle participe notamment au mécanisme moléculaire permettant la perméabilité de la barrière hémato-encéphalique (Juliano, R. L. & Ling, V. A surface glycoprotein modulating drug permeability in Chinese hamster ovary cell mutants. Biochim Biophys Acta 455, 152-162, doi:0005-2736(76)90160-7 [pii] (1976), [13]). Ainsi, plusieurs agents anticancéreux, comme la doxorubicine et les vinca-alcaloïdes sont activement rejetés hors des cellules par la P-gp ([5]). Ceci permet d'expliquer l'efficacité limitée de la chimiothérapie dans le traitement des tumeurs cérébrales. Au niveau des testicules, la P-gp permet la protection des cellules germinales contre les composés xénobiotiques. La présence de la P-gp au niveau du placenta témoigne de son rôle de régulateur du transfert de xénobiotiques de la mère au foetus ([8]). De nombreux médicaments sont des substrats de la P-gp (paclitaxel, daunorubicine, mitoxantrone par exemple). Cette protéine intervient également dans la biodisponibilité des médicaments ([9]).

La P-gp a récemment été associée aux développements de résistance de traitements antiviraux, en modulant la biodisponibilité orale des inhibiteurs de protéases et leur pénétration dans le système nerveux central (Kim, R. B. Drug transporters in HIV Therapy. Top HIV Med 11, 136-139 (2003), [15]). Le spectre de transport de ces pompes est très large, ce qui fait qu'elles sont capables d'effluer un grand nombre de composés, de nature variée, et utilisés par exemple pour traiter différentes pathologies, dont le cancer.

La MRP1 est une protéine polytopique transmembranaire, transporteur d'anions organiques conjugués comme le cystéinyl leucotriène (LTC(4)). Ce transporteur confère une résistance à la vincristine, aux anthracyclines et à l'étoposide. La MRP1 a une distribution tissulaire large, incluant la membrane basolatérale des cellules épithéliales de la plupart de tissus ; elle est exprimée à un niveau relativement élevé dans les poumons, les testicules et les reins. Une surexpression de la MRP1 a été observée dans les cancers du poumons, du sein, de la prostate, du col de l'utérus, et les leucémies traités par chimiothérapie.

La BCRP est présente dans la membrane plasmique des cellules. Cette protéine a été découverte dans le placenta (ALLIKMENTS R., SCHRIML L. M., HUTCHINSON A., ROMANO-SPICA V., DEAN M. Cancer Res., 1998, 58 (23), 5337-5339, [16]) puis dans le cancer du sein. Le taux de BCRP dans le placenta est 100 fois supérieur à celui retrouvé dans le cerveau, la prostate, le petit intestin, les testicules, les ovaires, le colon et le foie. La BCRP est associée à la multi-résistance de certaines cellules cancéreuses mais assure aussi différentes fonctions physiologiques. En effet, elle contribue à l'absorption, la distribution, le métabolisme et l'excrétion de certains xénobiotiques. D'autre part, la BCRP participerait significativement au transport des substances naturelles comme les polyphénols des plantes (COORAY H. C., JANVILISRI T., VAN VEEN H. W., HLADKY S. B., BARRAND M. A. Biochem. Biophys. Res. Commun., 2004, 317(1), 269-275, [17]) et à la protection du foetus contre certaines substances toxiques du sang maternel. La BCRP réduit l'absorption du topotécan (JONKER J. W., SMIT J. W., BRINKHUIS R. F., MALIEPAARD M., BEIJNEN J.H., SCHELLENS J. H. J. Natl. Cancer Inst., 2000, 92, 1651-1656, [18]) au niveau intestinal ; cette protéine intervient donc dans la pharmacocinétique et la biodisponibilité des médicaments.

Ces protéines d'efflux sont donc naturellement exprimées dans des tissus normaux afin d'expulser les toxines et les déchets produits par les cellules (e.g., poumon, rein et foie). Elles sont aussi produites au niveau de la barrière hémato encéphalique pour empêcher les toxines de pénétrer dans le cerveau.

Plusieurs études montrent l'impact de l'expression de ces transporteurs sur l'espérance de vie et de rémission des patients traités au moyen d'une chimiothérapie. La résistance à la chimiothérapie est notamment illustrée à la figure 1, tirée de l'étude publiée par Benderra et al. (Benderra, Z. et al. Breast Cancer Resistance Protein and P-glycoprotein in 149 Adult Acute Myeloid Leukemias. Clin Cancer Res 10, 7896-7902, doi:10.1158/1078-0432.ccr-04-0795 (2004) [12]), qui montre que l'espérance de vie et le pourcentage de rémission diminuent lorsque les patients atteints de leucémie myéloïde aiguë développent une surexpression de P-gp et/ou de BCRP.

Une des stratégies potentielles pour diminuer l'impact de l'expression de ces transporteurs sur le traitement chimiothérapeutique consiste à co-administrer l'agent chimiothérapeutique avec un inhibiteur de ces protéines d'efflux. Plusieurs molécules ont été développées dans ce but, et trois générations d'inhibiteurs sont actuellement connues. A cet égard, les tableaux 1a et 1 B montrent l'activité des inhibiteurs MDR sur les différents transporteurs ABC (Modok, S., Mellor, H. R. & Callaghan, R. Modulation of multidrug resistance efflux pump activity to overcome chemoresistance in cancer. Current Opinion in Pharmacology 6, 350-354 (2006), [19]). Les symboles indiquent soit que l'inhibiteur est actif (+) soit qu'il est inactif (-) sur un transporteur spécifié, ou que l'effet n'est pas connu (?).

Tableau 1a

| Transporteur | Seconde génération d'inhibiteurs | |
|---|---|---|
| | PSC833 (valspodar) | VX-710 (biricodar) |
| ABCB1 | + | + |
| ABCC1 | + | + |
| ABCG2 | - | + |

Tableau 1b

| Transporteur | Troisième génération d'inhibiteurs | | | | |
|---|---|---|---|---|---|
| | GF120918 (elacridar) | R101933 (laniquidar) | XR9576 (tariquidar) | Ly335979 (zosuquidar) | ONT-093 (ontogen) |
| ABCB1 | + | + | + | + | + |
| ABCC1 | - | ? | - | - | - |
| ABCG2 | + | ? | + | - | ? |

Certains inhibiteurs, comme le tariquidar (numéro Chemical Abstract Service 206873-63-4) par exemple, ont plusieurs cibles. Ceci n'est pas nécessairement un avantage puisque idéalement, un agent réversible, c'est-à-dire inhibiteur de protéine d'efflux, ne devrait cibler qu'une seule protéine d'efflux à la fois afin de préserver les fonctions physiologiques des autres dans les cellules saines. D'autres agents, tel que le zosuquidar (numéro Chemical Abstract Service 167354-41-8), ont été récemment stoppés en phase III à cause de leurs effets indésirables.

En effet, les protéines de transport ont des spectres d'efflux redondants, ce qui ralentit le développement de modulateurs de ces protéines car le modulateur d'un transporteur donné pouvant altérer le fonctionnement d'un autre transporteur.

De plus, il est important de préserver les fonctions physiologiques de ces transporteurs s'ils ne sont pas impliqués dans une résistance donnée.

Des di- ou tripeptides hydrophobes appelés réversines possèdent des propriétés inhibitrices de l'activité de la P-gp, et cela sans effet toxique aux doses efficaces utilisées (SARKADI B., SEPRŐDI J., CSUKA O., MAGOCSI M., MEZÔ I., PALYI I., TEPLÁN I., VADÁSZ Z., VINCZE, B. US 6,297,216 B1, 2 octobre 2001, [21], SEPRŐDI J., MEZÔ I., VADÁSZ Zs., SZABÓ K., SARKADI B., TEPLÁN I. in peptides 1996, Proceedings of the Twenty-Fourth European Peptide Symposium ; September 8-13, 1996, Edinburgh, Scotland. Robert RAMAGE and Roger EPTON (Eds), 1998, pp 801-802 [22]). En particulier, les réversines les plus actives sont les réversines 121, 1092 et 213. Ces dipeptides hydrophobes ont été étudiés par Sharom et al. (SHAROM F. J., YU X., LU P., LIU R., CHU J. W. K., SZABÔ K., MULLER M., HOSE C. D., MONKS A., VARADI A., SEPRŐDI J., SARKADI B. Biochem. Pharmacol., 1999, 58(4), 571-586 [23]) et se sont révélés très affins pour la P-gp, avec une constante de fixation sub-micromolaire et une constante d'inhibition de l'ordre du micromolaire.

Toutefois, même si le développement d'inhibiteurs des transporteurs ABC de type MDR est une voie majeure pour lutter contre la chimiorésistance des cancers, ce concept reste perfectible car il repose essentiellement sur la recherche de composés capables d'entrer en compétition avec le substrat au niveau du site de transport de ces protéines.

Le document de la Thèse d'A. Koubeissi (Thèse, Synthèse et étude biologique d'analogues di- et tripeptidiques de réversines susceptibles de moduler l'activité de deux protéines de transport, la glycorpotéine P et BCRP (2007), [24]) souligne que la contrainte majeure de cette approche est que les inhibiteurs ainsi sélectionnés peuvent devenir les substrats d'autres transporteurs MDR, capables d'adapter leur site de transport, intrinsèquement poly-spécifique. Dans ce contexte, ce document décrit la synthèse d'inhibiteurs analogues de ces réversines afin de préciser les requis structuraux nécessaires à l'activité vis-à-vis de la P-gp et la BCRP. Toutefois, ce document ne décrit pas ces analogues comme actifs ou susceptibles d'application dans le cadre d'un traitement chimiothérapeutique.

Ces exemples démontrent qu'il reste de réels besoins de fournir de nouveaux composés susceptibles d'être utilisés dans le cadre d'un traitement thérapeutique, qui se comportent comme des inhibiteurs spécifiques d'une pompe d'efflux et qui ne présentent pas une inhibition significative des pompes d'efflux qui ne sont pas impliquées dans la résistance en cause.

**Description de l'invention**

L'invention permet précisément de pallier les inconvénients de l'art antérieur et de répondre à ces besoins.

En effet, les inventeurs fournissent une famille de composés modulateurs ou des inhibiteurs de la P-gp pouvant être utilisés comme médicament, ou pour la préparation d'un médicament, utilisable notamment dans les traitements chimiothérapeutiques, ou dans des compositions pharmaceutiques utilisables chez un mammifère, notamment l'Homme ou l'animal.

Ainsi, la présente invention se rapporte à l'utilisation de composé de formule (I) éventuellement sous forme hydratée ou solvatée ou de l'un de ses sels pharmaceutiquement acceptables.

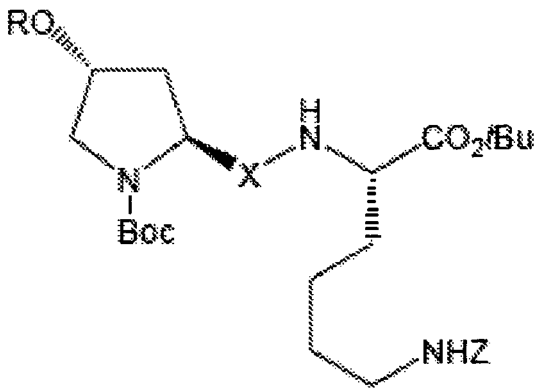

(I)

pour la préparation d'un médicament, dans laquelle :

- R représente un groupe Bn, -COBn, ou -COcHex, ou $-CH_2cHex$,
- X représente $-CH_2$ ou -CO,
- Z représente un groupe benzyloxycarbonyle.

Dans le cadre de l'invention, les abréviations suivantes sont utilisées : Boc pour *tert*-butyloxycarbonyle, Bn pour benzyle, cHex pour cyclohexyle, Hyp pour 4-hydroxyproline, Lys pour lysine, Z pour benzyloxycarbonyle, *t*Bu pour tert-butyle, *O* pour ortho, Ser pour sérine, Me pour méthyle.

En d'autres termes, l'invention se rapporte à un composé de formule (I) ou un de ses sels tel que défini ci-dessus, pour son utilisation comme médicament.

Il va de soi que les stéréoisomères, les mélanges ainsi que les sels du composé de formule (I) font partie de l'invention.

Le composé de formule (I) comme médicament présente l'avantage de ne pas induire une inhibition significative de la MRP1 et de la BCRP, préservant ainsi leurs fonctions physiologiques respectives.

Le composé de formule (I) comme médicament présente les avantages suivants : une spécificité de la P-gp, une affinité pour la P-gp meilleure que micromolaire (c'est-à-dire inférieure ou égale à 1 micromolaire), par exemple jusqu'à 7 fois meilleure que celle de la réversine 121 pour la P-gp, un niveau d'inhibition meilleur que celui de la réversine 121, peu ou pas d'effets indésirables et une absence de compétitivité avec le ou les substrats transportés par la pompe.

Quand X représente $-CH_2$, R peut être choisi parmi -Bn, -COBn, ou -COcHex, ou $-CH_2cHex$.

Par exemple, X peut représenter $CH_2$ et R peut représenter Bn.

Quand X représente -CO, R peut être choisi parmi -Bn, -COBn, ou - COcHex, ou $-CH_2cHex$.

Par exemple, X peut représenter -CO et R peut représenter -COBn.

Dans un autre exemple, X peut représenter -CO et R peut représenter COcHex.

Dans un autre exemple, X peut représenter -CO et R peut représenter Bn.

Dans un autre exemple, X peut représenter -CO et R peut représenter $CH_2cHex$.

Le composé peut avoir une des formules choisies parmi les formules (II), (III), (IV), (V), ou (VI) ci-après désignée respectivement par « composé No. 6 », « composé No. 2 », « composé No. 3 », « composé No. 4 » et « composé No. 5 » :

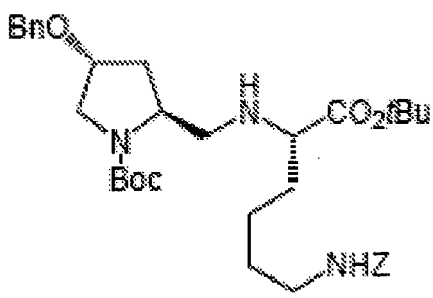

(II)

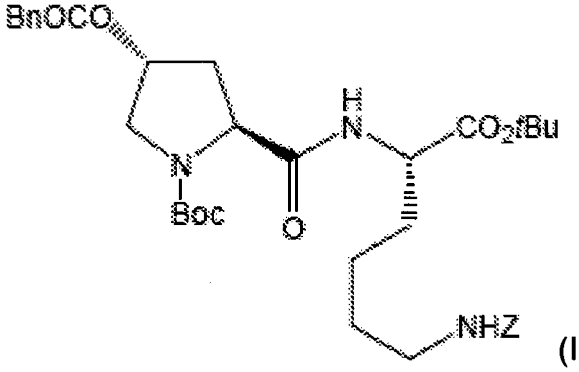

(III)

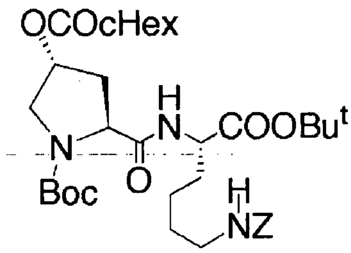

(IV)

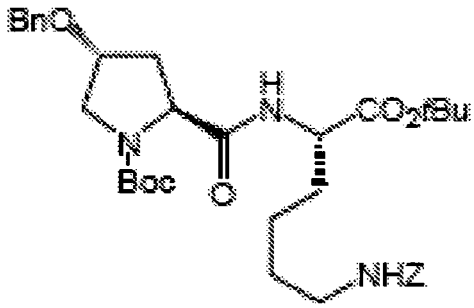

(V)

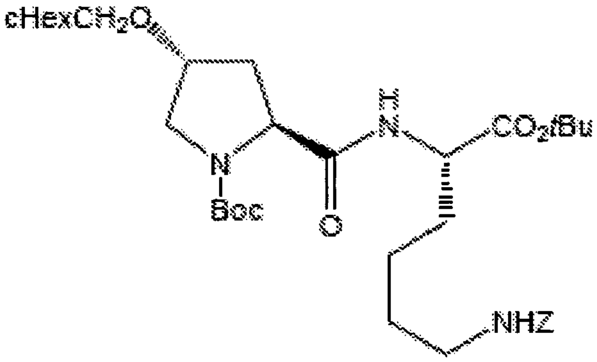

(VI)

Les composés de formules ci-dessus comprennent également ceux dans lesquels un ou plusieurs atomes d'hydrogène, de carbone ou d'halogène, notamment de fluor ou de chlore ont été remplacés par leur isotope radioactif par exemple le tritium ou le carbone 14.

L'ensemble de ces composés présente les propriétés suivantes : une absence d'inhibition significative de la MRP1 et de la BCRP, une spécificité vis-à-vis de la P-gp, une affinité pour la P-gp meilleure que micromolaire (c'est-à-dire inférieure ou égale à 1 micromolaire), par exemple jusqu'à 7 fois meilleure que celle de la réversine 121 pour la P-gp, un niveau d'inhibition meilleur que celui de la réversine 121, peu ou pas d'effets indésirables et une absence de compétitivité.

L'invention s'entend d'une composition comprenant un seul type de composé de formule précédemment définie,

ou un mélange d'au moins deux de ces composés.

Dans le cas où les composés sont sous forme de mélange, les proportions de chacun des composés seront déterminées par l'homme du métier, par exemple en fonction du mode d'administration choisi.

Le mélange de ces composés présente les propriétés suivantes : une absence d'inhibition significative de la MRP1 et de la BCRP, une spécificité vis-à-vis de la P-gp, une affinité pour la P-gp meilleure que micromolaire (c'est-à-dire inférieure ou égale à 1 micromolaire), par exemple jusqu'à 7 fois meilleure que celle de la réversine 121 pour la P-gp, un niveau d'inhibition meilleur que celui de la réversine 121, peu ou pas d'effets indésirables et une absence de compétitivité.

Les composés de formule définie précédemment peuvent être sous forme d'isomères purs ou sous forme de mélange d'isomères.

Le mélange de ces isomères peut être déterminé par l'homme du métier, par exemple en fonction du mode d'administration choisi.

Le mélange de ces isomères présente l'avantage de ne pas induire une inhibition significative de la MRP1 et de la BCRP.

Le mélange de ces isomères présente les avantages suivants : une spécificité de la P-gp, une affinité pour la P-gp meilleure que micromolaire (c'est-à-dire inférieure ou égale à 1 micromolaire), par exemple jusqu'à 7 fois meilleure que celle de la réversine 121 pour la P-gp, un niveau d'inhibition meilleur que celui de la réversine 121, peu ou pas d'effets indésirables et une absence de compétitivité.

Les sels des composés de formule définie précédemment comprennent ceux avec des acides minéraux ou organiques, et les sels pharmaceutiquement acceptables.

En tant qu'acide approprié on peut citer l'acide oxalique ou un acide optiquement actif, par exemple un acide tartrique, un acide dibenzoyltartrique, un acide mandélique ou un acide camphosulfonique, et ceux qui forment des sels physiologiquement acceptables, tels que le chlorhydrate, le bromhydrate, le sulfate, l'hydrogénosulfate, le dihydrogénosulfate, le maléate, le fumarate, le 2-naphtalènesulfate, le paratoluènesulfonate, le mésylate, le bésylate, l'isotionate.

Le mélange de ces isomères présente l'avantage de ne pas induire une inhibition significative de la MRP1 et de la BCRP.

Le mélange de ces isomères présente les avantages suivants : une spécificité de la P-gp, une affinité pour la P-gp meilleure que micromolaire (c'est-à-dire inférieure ou égale à 1 micromolaire), par exemple jusqu'à 7 fois meilleure que celle de la réversine 121 pour la P-gp, un niveau d'inhibition meilleur que celui de la réversine 121, peu ou pas d'effets indésirables et une absence de compétitivité.

En tant que composé sous forme hydratée, on peut citer, à titre d'exemple, les semihydrates et monohydrates.

Les composés de l'invention peuvent être préparés par tout procédé connu de l'homme du métier. On peut par exemple utiliser les procédés décrits dans le document A. Koubeissi ([24]).La synthèse des composés de l'invention peut être réalisée par couplage peptidique du dérivé protégé de la trans-4-hydroxy-L-proline avec la H-Lys(Z)-O*t*Bu.HCl commerciale par la méthode de l'anhydride mixte (Lai, M. Y. H. et al. Synthesis and pharmacological evaluation of glycine-modified analogues of the neuroprotective agent glycyl-l-prolyl-l-glutamic acid (GPE). Bioorganic & Medicinal Chemistry 13, 533-548 (2005), [25]). L'analogue aminométhylénique composé No. 6 peut être obtenu par amination réductrice dans des conditions classiques (Martinez, J. et al. Synthesis and biological activities of some pseudo-peptide analogs of tetragastrin: the importance of the peptide backbone. Journal of Medicinal Chemistry 28, 1874-1879 (1985), [26]) à partir de l'aminoaldéhyde correspondant dérivé de la trans-4-hydroxy-L-proline protégé et de la H-Lys(Z)-O*t*-Bu.HCl.

La purification de ces composés peut être réalisée par toute technique connue de l'homme du métier (W. Clark Still, Michael Kahn, and Abhijit Mitra J. Org. Chem. Rapid Chromatographic Technique for Preparative Separations with Moderate Resolution, J. Org. Chem., 1978, 43(14), 2923-2925 [14]).

Les sels peuvent être préparés selon des techniques bien connues de l'homme du métier (Vogel's textbook of practical organic chemistry, Ve édition, 1989, Longman Scientific & Technical éd. [34]).

La réversine 121 est un composé de formule (VII) :

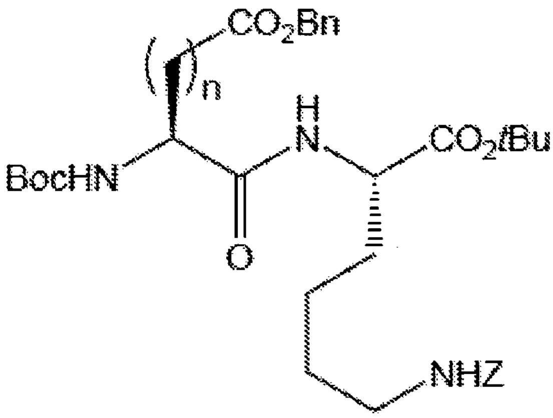

(VII)

La réversine peut être préparée par tout procédé connu de l'homme du métier, par exemple celui décrit dans le document US6,297,216, ou dans les documents Sharom et al. ([23]) ou Palyi et al. (Palyi, I. et al. Compounds for reversing drug resistance. Hungary patent (2001), [27]).

Les composés tels que définis ci-avant présentent une valeur faible d'$IC_{50}$. L'$IC_{50}$ est définie comme la concentration de composé bloquant ou inhibant 50% de l'efflux d'agent chimiothérapeutique.

Cette valeur peut être inférieure à celle de la réversine 121. Elle est par exemple comprise entre $0{,}01\pm0{,}001$ $\mu$M et $1{,}5\pm0{,}03$ $\mu$M. Par exemple, le composé tel que précédemment défini à une $IC_{50}$ d'environ $0{,}22\pm0{,}03$ $\mu$m.

Les tests permettant de déterminer l'activité des composés de l'invention et celle de la réversine 121 sont bien connus de l'homme du métier. Il est possible d'utiliser par exemple les tests décrits dans l'exemple 2 ci-après.

Les composés de l'invention présentent une inhibition non significative de la MRP1 et de la BCRP. Par exemple, les composés de l'invention peuvent présenter une inhibition de la MRP1 ou de la BCRP, à 10 $\mu$M, inférieure à 15%.

Avantageusement, aucun signe de cytotoxicité n'est observé avec les composés de l'invention aux doses pharmacologiquement actives, ce qui les rend compatibles avec leur utilisation comme médicaments.

On entend par « médicament », au sens de la présente invention, toute substance ou composition pouvant être utilisée chez un mammifère, i.e. l'Homme ou l'animal, ou pouvant leur être administrée, en vue d'établir un diagnostic médical ou de restaurer, corriger ou modifier leurs fonctions physiologiques en exerçant une action pharmacologique, immunologique ou métabolique. Il peut s'agir d'un composé ou composition de l'invention administré en tant qu'adjuvant d'un traitement chimiothérapeutique d'un patient, pour lequel l'organisme du patient traité développe une résistance. Le médicament selon l'invention peut être destiné à restaurer ou maintenir l'activité du traitement chimiothérapeutique. Le médicament selon l'invention peut permettre de bloquer, moduler ou inhiber la P-gp. Le médicament selon l'invention peut permettre d'empêcher ou de diminuer l'efflux d'agent chimiothérapeutique hors des cellules cibles du traitement chimiothérapeutique. Les composés de l'invention sont donc utilisés en tant que médicament adjuvant d'un agent chimiothérapeutique.

On entend par « animal », au sens de la présente invention les animaux domestiques, par exemple le chien, les félidés comme le chat et les félins, le lapin, les bovins, les ovins, les équidés, les mammifères, les rongeurs, les oiseaux, les reptiles, les sauriens, cette liste n'étant pas limitative.

On entend par « mammifère », au sens de la présente invention, tout animal vertébré à sang chaud, dont le coeur possède quatre cavités. Il peut s'agir par exemple de l'Homme, des chiens, chats, des rongeurs, des bovins, des équidés, des ovins, cette liste n'étant pas limitative.

Le composé de l'invention peut être administré en une quantité efficace au mammifère. Par « quantité efficace » on entend, au sens de la présente invention, toute quantité d'un composé de l'invention permettant d'obtenir l'effet recherché.

Cette quantité peut permettre d'améliorer un ou plusieurs de paramètres caractéristiques de la pathologie traitée. Cette quantité peut en outre permettre d'obtenir une réduction totale ou partielle de l'activité d'efflux de la P-gp. Par exemple, une quantité efficace du composé de l'invention peut permettre d'obtenir une réduction d'au moins 50%, et avantageusement au moins 60%, ou 70%, ou 80%, ou 90% ou 99% de l'activité d'efflux de la P-gp.

En d'autres termes, cette quantité peut permettre de diminuer la résistance à une substance active, notamment à un agent chimiothérapeutique.

Avantageusement, la quantité efficace de composé de structure (I) peut être comprise entre 0,01 et 100 $\mu$M. Alternativement, la quantité efficace peut être comprise entre 1 et 100 mg par kg de poids du corps et par jour.

Le médicament peut donc être destiné à réduire l'activité de la protéine d'efflux ABCB1 chez un mammifère.

Le médicament peut être destiné à diminuer la résistance à une substance active du point de vue pharmaceutique.

Le composé de l'invention peut être administré seul, ou avec une substance active du point de vue pharmaceutique.

Dans le cas où il est administré avec une substance active du point de vue pharmaceutique, il peut être qualifié d'adjuvant à un traitement par cette substance.

Par exemple, le médicament peut être un adjuvant au traitement ch imiothérapeutique d'un cancer ou d'une infection.

Par « cancer » on entend, au sens de la présente invention, toute condition pathologique impliquant une croissance anormale/dérégulée des cellules au sein d'un tissu d'un organisme vivant. A titre d'exemple on peut citer les carcinomes, les lymphomes, comme la Maladie de Hodgkin et le lymphome non-hodgkinien, les blastomes, les sarcomes, les leucémies, et plus particulièrement le cancer pulmonaire, le cancer du sein, le cancer du côlon : cancer du côlon, du rectum, du pancréas, le myélome multiple, notamment le cancer de la moelle osseuse, le sarcome de Kaposi, le cancer des testicules, l'adénocarcinome, le mésothéliome, le gliome, le mélanome, le cancer du rein, de la prostate, l'hépatocarcinome, cette liste n'étant pas limitative.

Par « infection » ou « maladie infectieuse » on entend, au sens de la présente invention, toute condition pathologique impliquant l'invasion de cellules au sein d'un tissu par un organisme vivant. Cet organisme vivant peut être un virus, une bactérie, un parasite, un champignon. A titre d'exemple on peut citer le tétanos, le paludisme, les pneumonies, la grippe, le sida, les septicémies, les cardiopathies rhumatismales, les appendicites, les péritonites, les tuberculoses, les infections intestinales, les hépatites virales, cette liste n'étant pas limitative.

Par « substance active du point de vue pharmaceutique », au sens de la présente invention, on entend toute substance ayant un effet pharmacologique lorsqu'il est administré à un Homme ou à un animal.

Par exemple, une substance active du point de vue pharmaceutique peut être un agent chimiothérapeutique, par exemple antibiotique, antifongique, anticancéreux, anti-infectieux, mais aussi toute molécule d'origine chimique.

Par « agent chimiothérapeutique », au sens de la présente invention, on entend toute substance chimique ou synthétique utilisée dans le cadre d'une thérapie. Notamment, il peut s'agir d'une substance active destinée à détruire les cellules cancéreuses ou infectées par un agent pathogène. Il peut s'agir alors d'agents anti-cancéreux ou d'agents anti-infectieux. On peut citer à titre d'exemple les anthracyclines, les inhibiteurs de topoisomérase, les antimétaboliques comme les antifolates, les inhibiteurs de tyrosine kinase, les antiviraux comme par exemple les inhibiteurs de transcriptase inverse, les antiparasitaires, les antifongiques, et notamment la daunorubicine, la doxorubicine, la mitoxantrone, la camptotécine et ses dérivés, l'irinotécan, le topotécan, les indolocarbazoles, le méthotrexate, l'imatinib, le gefitinib, la zidovudine, la lamivudine, l'abacavir, l'ivermectine, l'albendazole, l'oxfendazole, le kétoconazole, cette liste n'étant pas limitative.

Dans le cas où le composé est administré seul, aucun agent chimiothérapeutique n'est administré.

Dans le cas où le composé est administré avec un agent chimiothérapeutique, le composé de l'invention et l'agent chimiothérapeutique peuvent être administrés de manière simultanée, séquencée, successive ou étalée dans le temps.

Les composés de l'invention peuvent être utilisés pour potentialiser l'effet, c'est-à-dire augmenter l'effet, d'agents chimiothérapeutiques, notamment d'agents anti-infectieux ou anti-cancéreux. Par exemple, ces agents chimiothérapeutiques peuvent devenir peu ou pas actifs vis-à-vis de souches résistantes via un mécanisme d'efflux. Par « potentialisation », on entend, au sens de la présente invention, qu'en associant un composé de formule (I) et un agent chimiothérapeutique, par exemple un agent anti-infectieux ou anti-cancéreux, on obtient un effet thérapeutique supérieur à celui obtenu avec l'un ou l'autre seulement des composés. Avantageusement, l'effet thérapeutique peut être supérieur à la somme des effets obtenus séparément.

Une quantité efficace de l'agent chimiothérapeutique est de préférence administrée. Avantageusement, la quantité efficace est une quantité ne permettant pas d'obtenir la destruction des cellules cibles quand le composé de l'invention n'est pas administré avec l'agent chimiothérapeutique. La quantité efficace de l'agent thérapeutique peut être inférieure à la quantité efficace de l'agent chimiothérapeutique lorsque l'agent est administré sans le composé de l'invention. Avantageusement, la dose d'agent chimiothérapeutique administré peut permettre d'obtenir une destruction totale ou partielle des cellules cibles. Avantageusement, une quantité efficace de l'agent chimiothérapeutique peut permettre d'obtenir une destruction d'au moins 50%, et avantageusement au moins 60%, ou 70%, ou 80%, ou 90% ou 99% des cellules cibles. Avantageusement, la quantité efficace de composé de l'agent chimiothérapeutique peut être comprise entre 0,1 et 100 $\mu$M par rapport à la composition totale. Alternativement, la quantité efficace peut être comprise entre 0,01 et 100 mg par kg de poids du corps et par jour.

Le composé de l'invention et l'agent chimiothérapeutique peuvent être administrés dans un rapport permettant d'obtenir une inhibition totale ou partielle de l'activité d'efflux de la P-gp et une destruction totale ou partielle des cellules cibles.

Différents ordres d'administration ou de traitement peuvent être prévus. Le composé de l'invention peut être administré avant, par exemple 5 minutes, 15 minutes, 30 minutes, 45 minutes, 1 heure, 2 heures, 4 heures, 6 heures,

12 heures, 24 heures, 48 heures, 72 heures, 96 heures, 1 semaine, 2 semaines, 3 semaines, 4 semaines, 5 semaines, 8 semaines, ou 12 semaines avant l'administration de l'agent chimiothérapeutique.

Alternativement, le composé de formule telle que définie précédemment peut être administré de manière concomitante, ou après l'administration de l'agent de chimiothérapie, par exemple 5 minutes, 15 minutes, 30 minutes, 45 minutes, 1 heure, 2 heures, 4 heures, 6 heures, 12 heures, 24 heures, 48 heures, 72 heures, 96 heures, 1 semaine, 2 semaines, 3 semaines, 4 semaines, 5 semaines, 8 semaines, ou 12 semaines après l'administration de l'agent chimiothérapeutique.

Un autre objet de l'invention se rapporte au composé de formule telle que définie précédemment, ses mélanges, ses sels, ses isomères, comme médicament. Ce médicament peut être utilisé chez l'Homme ou chez l'animal.

L'invention se rapporte également au composé de formule telle que précédemment définie pour le traitement d'un cancer ou d'une infection. Ce composé peut être utilisé chez l'Homme ou chez l'animal.

L'invention s'entend également du composé de formule telle que définie précédemment pour réduire l'activité de la protéine d'efflux ABCB1 chez un Homme ou un animal.

Un autre objet de l'invention se rapporte à une composition pharmaceutique comprenant le composé de l'invention.

La concentration du composé de l'invention peut être comprise, dans la composition pharmaceutique, entre 0,001 à 500 $\mu$M. Par exemple, la concentration est comprise entre comprenant 0,001 et 50 $\mu$M, ou entre 0,001 et 1 $\mu$M.

Le composé de l'invention et l'agent chimiothérapeutique peuvent être administrés, séparément, chacun dans une composition pharmaceutique distincte.

Alternativement, le composé de l'invention et l'agent chimiothérapeutique peuvent être administrés dans une composition pharmaceutique unique. En d'autres termes, le médicament de l'invention peut se présenter sous la forme d'une composition pharmaceutique unique combinant, dans une même formulation, (i) le composé de l'invention et (ii) un agent chimiothérapeutique tel que défini précédemment. Une telle composition pharmaceutique peut être utilisée chez l'Homme ou chez l'animal.

La composition pharmaceutique comprend en outre un support pharmaceutiquement acceptable, avantageusement choisi selon la forme pharmaceutique et le mode d'administration souhaité. Les supports pharmaceutiquement acceptables pouvant être utilisés sont ceux connus de l'homme du métier. On peut citer à titre d'exemple la gélatine, l'amidon, le lactose, le stéarate de magnésium, le talc, la gomme arabique ou tout analogue.

La composition pharmaceutique présente l'avantage de ne pas induire une inhibition significative de la MRP1 et de la BCRP.

La composition pharmaceutique présente les avantages suivants : une spécificité de la P-gp, une affinité pour la P-gp meilleure que micromolaire (c'est-à-dire inférieure ou égale à 1 micromolaire), par exemple jusqu'à 7 fois meilleure que celle de la réversine 121 pour la P-gp, un niveau d'inhibition meilleur que celui de la réversine 121, peu ou pas d'effets indésirables et une absence de compétitivité.

Le mode d'administration de la composition pharmaceutique ou du médicament peut être oral, sublingual, sous-cutané, intramusculaire, intraveineux, topique, intratrachéal, intranasal, transdermique, rectal ou intraoculaire.

La forme d'administration de la composition ou du médicament comprend toutes les formes compatibles avec le but recherché. Ces formes d'administration sont connues de l'homme du métier, et comprennent les comprimés, par exemple les comprimés pelliculés, enrobés, sécables, les gélules, les poudres, les granules, les suspensions ou solutions orales, les crèmes, les pommades, les lotions ou les collyres, les sirops, les émulsions, cette liste n'étant pas limitative.

La forme du médicament ou de la composition pharmaceutique peut être une forme adaptée à une activité prolongée ou retardée. La forme peut être adaptée à une libération continue d'une quantité prédéterminée de composé de l'invention et éventuellement d'agent chimiothérapeutique.

Les formulations, notamment les comprimés, les gélules, les granules, peuvent être enrobées de saccharose, ou d'un dérivé cellulosique.

Par exemple, les gélules peuvent être préparées en mélangeant l'ingrédient actif avec un diluant et en versant le mélange obtenu dans des gélules, par exemple des gélules molles ou dures.

Les formes liquides contenant le médicament ou la composition pharmaceutique de l'invention peuvent avoir comme support tout liquide approprié. De tels supports sont connus de l'homme du métier, et l'on peut citer comme exemple l'eau, les solvants, notamment les solvants organiques tels le glycérol ou les glycols, ainsi que leurs mélanges, dans des proportions variées.

Une préparation sous forme de sirop, d'élixir ou de goutte peut contenir un édulcorant, par exemple acalorique, un antiseptique comme du méthylparabène ou du propylparabène, un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, ou avec des édulcorants ou des correcteurs de goût.

Un autre objet de l'invention est l'utilisation du composé de l'invention pour le traitement de pathologies. On peut citer à titre d'exemple les cancers ou les infections, chez l'Homme ou l'animal.

Un autre objet de l'invention se rapporte à l'utilisation d'un composé de formule (I) pour inhiber *in vitro* la pompe d'efflux ABCB1. Par exemple, un composé de formule (I) peut être utilisé pour cribler des molécules, notamment des agents chimiothérapeutiques.

Un autre objet de l'invention se rapporte à une méthode de traitement thérapeutique, notamment à un patient en présentant le besoin, comprenant l'administration d'un composé de structure (I) ou un de ses sels pharmaceutiquement acceptable.

D'autres avantages pourront encore apparaître à l'homme du métier à la lecture des exemples ci-dessous, illustrés par les figures annexées, donnés à titre illustratif.

## Brève description des figures

- La figure 1 montre l'impact de l'expression de la P-gp et de BCRP sur les pourcentages d'espérance de vie et de rémission de leucémies myéloïdes aigues ([12]).
- La figure 2 montre les composés No. 1, 2, 3, 4, 5 et 6 et leur effet sur la fonction d'efflux de la P-gp. La réversine 121 est le composé de référence ([23], [27]). Les pourcentages d'inhibition de la P-gp sont indiqués ainsi que la concentration de ½ effet maximal $IC_{50}$.
- Les figures 3A, B, C, D et E montrent l'inhibition (en pourcentage) de l'efflux de mitoxantrone (MTX), anticancéreux transporté par la P-gp respectivement par la réversine 121 ([27]), le composé No. 3, le composé No. 5, le composé No. 3, le composé No. 4, le composé No. 3, le composé No. 6. Chaque valeur d'$IC_{50}$ est obtenue par ajustement mathématique des points expérimentaux et reportée dans le tableau 2.
- La figure 4 montre la chimiosensibilisation à la mitoxantrone des cellules NIH3T3 contrôle (cercles, carrés) ou exprimant la P-gp (triangles, losanges) par le composé No. 6. Des concentrations (μM) croissantes en mitoxantrone (MTX) sont appliquées à des cellules NIH3T3 exprimant la P-gp ou n'exprimant pas la P-gp, en présence ou en absence du composé No. 6. Le taux de survie cellulaire est exprimé en pourcentage.
- Les figure 5A et 5B montrent respectivement la cytotoxicité des composés No. 4 et No. 6, appliquée à des concentrations croissantes (μM) à des cellules NIH3T3 exprimant (carrés) ou n'exprimant pas (cercles) la P-gp.
- Les figures 6A et 6B montrent les cinétiques d'inhibition de l'efflux de daunorubicine réalisé par la P-gp par le composé No. 6. La fluorescence intracellulaire de la daunorubicine est mesurée en présence de concentrations croissantes de No. 6, respectivement 0 (cercles), 0,12 μM (triangles), 0,22 μM (carrés), 0,44 μM (losanges) et 10 μM (triangles vers le haut), correspondant à 0, 25, 50, 75 et 100 % d'efficacité de l'inhibition d'efflux. Figure 6A : représentation directe, figure 6B, représentation en double inverse de Lineweaver & Burke.

## EXEMPLES

### EXEMPLE 1 : PRÉPARATION DES COMPOSÉS No. 2, 3, 4, 5 et 6

Dans les exemples qui suivent, les abréviations suivantes sont utilisées : h signifie heure, t.a. signifie température ambiante, asym signifie asymétrique, AcOEt signifie acétate d'éthyle, Boc signifie *tert*-butyloxycarbonyle, $(Boc)_2O$ signifie dicarbonate de di-tert-butyle, Bu signifie butyle, DMF signifie N,N-diméthylformamide, EP signifie éther de pétrole, ESI signifie *ionisation* électrospray pour « Electrospray Ionization », Et signifie éthyle, $Et_2O$ signifie diéthyle éther, HR signifie haute résolution, Me signifie méthyle, Ph signifie phényle, éq signifie équivalent, Hyp signifie 4-hydroxyproline, Lys signifie lysine, IR signifie infrarouge, RMN signifie résonance magnétique nucléaire, SM signifie spectrométrie de masse, TBTU signifie tétrafluoroborate de 2-(1-hydroxy-benzotriazol-1-yl)-1,1,3,3-Tétrahydrouronium.

#### 1) Synthèse du $N^{\alpha}$-Boc-*trans*-4-Hyp(COR)-Lys(Z)-O*t*Bu

La synthèse est réalisée à partir de la *trans*-4-hydroxy-L-proline commerciale (Novabiochem Réf. 04-10-0020) dont les fonctions acide et amine sont préalablement protégées. Ensuite, l'estérification de l'alcool, suivie de la déprotection de l'acide et couplage avec le dérivé diprotégé de la lysine commerciale) (Novabiochem Réf. 04-12-5122), conduit à l'analogue contraint cible.

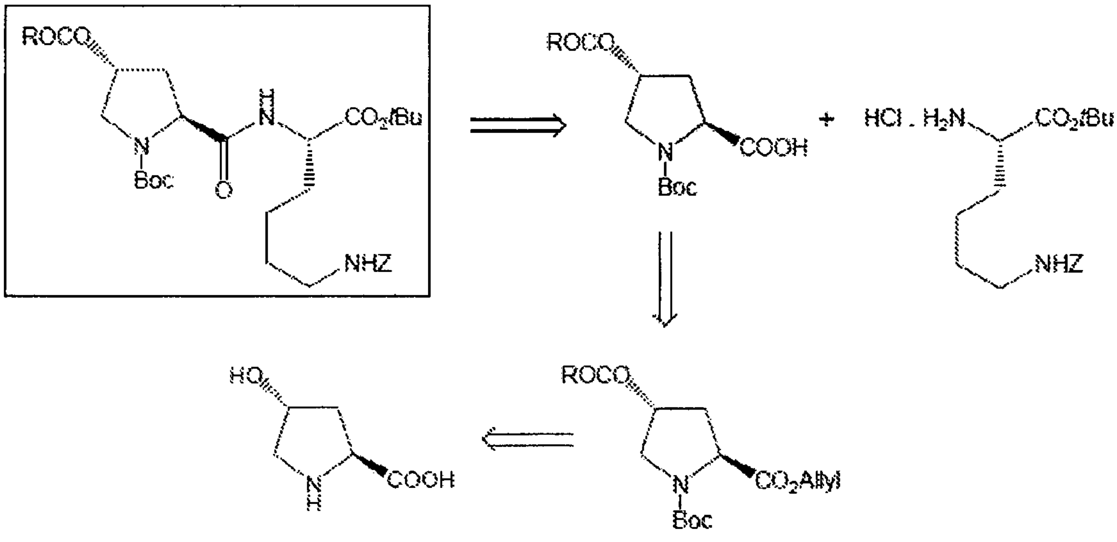

4-hydroxy-proline

**1.1) Synthèse de la *N*α-Boc-*trans*-4-Hyp-OAllyl**

Le dérivé 74 est préparé avec un rendement de 97 % selon le mode opératoire décrit par Carrasco et Brown (CARRASCO M. R., BROWN R. T. J. Org. Chem., 2003, 68(23), 8853-8858, [31]) sur la L-homosérine.

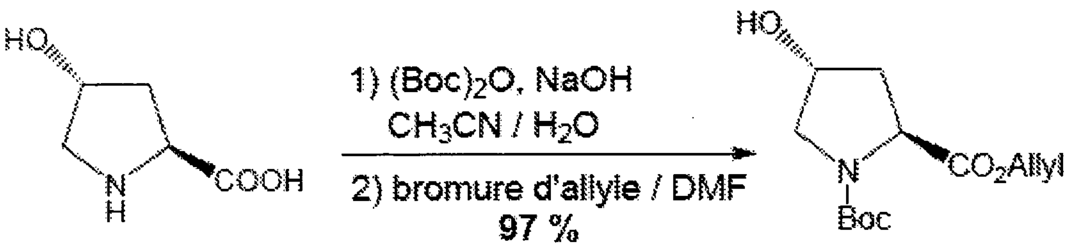


**74**

**Schéma 1 :** synthèse de la *N*α-Boc-*trans*-4-Hyp-OAllyl

**Ester allylique de l'acide (4*R*)-hydroxy-*N*-(*tert*-butyloxycarbonyl)-pyrrolidin-(2*S*)-oïque 74**

500 mg de *trans*-4-hydroxy-L-proline commerciale (3,816 mmol; 500 mg) et 170 mg de NaOH (4,2 mmol ; 1,1 éq) sont dissous dans 5 mL d'eau et 5 mL de $CH_3CN$. Ensuite 1,24 g de $(Boc)_2O$ (5,7 mmol ; 1,5 éq) est ajouté et la solution est agitée pendant la nuit à température ambiante. Les solvants sont évaporés et le résidu huileux obtenu est trituré avec de l'$Et_2O$, séché sous vide puis dissous dans 12 mL de DMF. La solution ainsi obtenue est traitée avec 0,364 mL de bromure d'allyle (4,2 mmol ; 1,1 éq) et la réaction est agitée pendant la nuit à température ambiante. Le DMF est évaporé et le résidu obtenu est dissous dans l'AcOEt. Cette phase organique est lavée trois fois avec 25 mL d'une solution saturée de $NaHCO_3$, une fois avec 25 mL d'eau, deux fois avec 25 mL d'une solution de $KHSO_4$ 0,1 M et une fois avec 25 mL d'une solution saturée de NaCl. La phase organique est séchée sur du $Na_2SO_4$ anhydre, filtrée et concentrée. Le produit (3,69 mmol ; 1 g) est obtenu sous la forme d'une huile incolore qui ne nécessite pas de purification.

**IR (NaCl ; film)** : 3436 ($\nu_{OH}$) ; 2978 et 2936 ($\nu_{CH}$) ; 1748 et 1682 ($\nu_{C=O}$) ; 1415 ($\nu_{C\text{-}C\text{-}Oasym}$) ; 1368 ($\nu_{CH3}$ *t*-Bu) ; 1159 ($\nu_{O\text{-}C\text{-}C\ asym}$).

**RMN $^1$H ($CDCl_3$ ; 300 MHz)** : 1,42 et 1,46 (9H, 2s, C($C\underline{H}_3$)$_3$ Boc 1er et 2ème conf.) ; 2,09 (1 H, m, $C\underline{H}_\beta$) ; 2,31 (1 H, m, $C\underline{H}_\beta$) ; 3,55 (2H, m, $C\underline{H}_2N$) ; 4,40-4,51 (2H, m, $C\underline{H}_\alpha$ et C$\underline{H}$OH) ; 4,65 (2H, d, J = 5,6 Hz, $C\underline{H}_2$-CH=$CH_2$) ; 4,26 (1 H, dd, J = 0,7 Hz et J = 10,1 Hz, $CH_2$-CH=$C\underline{H}_2$) ; 5,84 (1 H, dd, J = 0,9 Hz et J = 18,2 Hz, $CH_2$-CH=$C\underline{H}_2$) ; 5,91 (1 H, m, $CH_2$-C$\underline{H}$=$CH_2$).

**1.2) Synthèse de la $N^{\alpha}$-Boc-*trans*-4-Hyp(COR)-OAllyl**

L'alcool **74** est estérifié par le chlorure de phénylacétyle ou de cyclohexanoyle. La réaction est totale dans le cas du composé **75a** en utilisant un excès de chlorure de cyclohexanoyle et 2 équivalents de triéthylamine. Les deux esters **75a** et **75b** sont obtenus avec des rendements de 43 et 84 % respectivement.

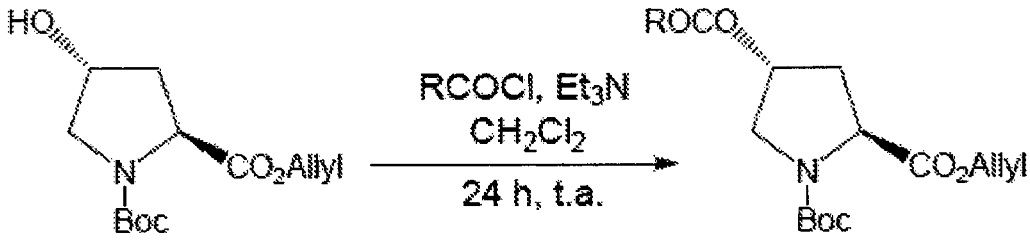


**74**

**75a** R = Bn **43 %**

**75b** R = cHex **84 %**

**Schéma 2 :** synthèse de la $N^{\alpha}$-Boc-*trans*-4-Hyp(COR)-Oallyl

**Ester allylique de l'acide (4R)-(benzylcarbonyloxy)-N-(tert-butyloxy-carbonyl)-pyrrolidin-(2*S*)-oïque 75a**

À une solution de $N^{\alpha}$-Boc-*trans*-4-Hyp-OAllyl **74** (0,738 mmol ; 200 mg) dans 7 mL de $CH_2Cl_2$, sont ajoutés 156 μL de triéthylamine (1,107 mmol ; 1,5 éq) et 120 μL de chlorure de phénylacétyle (0,885 mmol ; 1,2 éq) à 0°C. Après agitation pendant la nuit à température ambiante, 15 mL d'eau sont ajoutés et la réaction est extraite avec 20 mL de $CH_2Cl_2$. La phase organique est lavée avec de l'eau, séchée sur du $Na_2SO_4$ anhydre, filtrée et concentrée. Le résidu huileux jaune obtenu est purifié par chromatographie sur colonne de gel de silice (AcOEt / EP 20 : 80). Le produit (0,320 mmol ; 123 mg) est obtenu sous la forme d'une huile jaunâtre.

**IR (NaCl ; film) :** 3065 et 3031 ($\nu\varphi_{CH}$); 2978 et 2933 ($\nu_{CH}$) ; 1744 et 1704 ($\nu_{C=O}$) ; 1455 ($\nu\varphi_{C=C}$); 1403 et 1367 ($\delta_{CH3}$ *t*-Bu) ; 1258 ($\nu_{C\text{-}C\text{-}O\ asym}$) ; 1157 ($\nu_{O\text{-}C\text{-}C\ asym}$) ; 931 ($\delta_{CH}$ vinyl).
**RMN $^1$H ($CDCl_3$ ; 300 MHz) :** 1,44 et 1,46 (9H, 2s, $C(C\underline{H}_3)_3$ Boc 1er et 2ème conf.) ; 2,20 (1 H, m, $C\underline{H}_\beta$) ; 2,38 (1 H, m, $C\underline{H}_\beta$) ; 3,62-3,76 (4H, s+m, $C\underline{H}_2$Ph et $C\underline{H}_2$N) ; 4,32 et 4,43 (1 H, 2t, J = 8,0 Hz et J = 7,7 Hz, $C\underline{H}_\alpha$ 1er et 2ème conf.) ; 4,65 (2H, m, $C\underline{H}_2$-CH=$CH_2$) ; 5,22-5,38 (3H, m, $CH_2$-CH=$C\underline{H}_2$ et C$\underline{H}$OCOBn) ; 5,92 (1 H, m, $CH_2$-C$\underline{H}$=$CH_2$) , 7,33 (5H, m, Ph).

**Ester allylique de l'acide (4R)-(cyclohexylcarbonyloxy)-*N*-(*tert*-butyloxy-carbonyl)-pyrrolidin-(2*S*)-oïque 75b**

À une solution de $N^{\alpha}$-Boc-*trans*-4-Hyp-OAllyl **74** (0,960 mmol ; 260 mg) dans 8 mL de $CH_2Cl_2$, sont ajoutés 270 μL de triéthylamine (1,92 mmol ; 2 éq) et 224 μL de chlorure de cyclohexanoyle (1,632 mmol ; 1,7 éq) à 0°C. Après agitation pendant la nuit à température ambiante, 15 mL d'eau sont ajoutés et la réaction est extraite avec 20 mL de $CH_2Cl_2$. La phase organique est lavée avec de l'eau, séchée sur du $Na_2SO_4$ anhydre, filtrée et concentrée. Le résidu huileux jaunâtre obtenu est purifié par chromatographie sur colonne de gel de silice (AcOEt / EP 20 : 80). Le produit (0,808 mmol ; 308 mg) est obtenu sous la forme d'une huile incolore.
**IR (NaCl ; film) :** 2977 et 2934 ($\nu_{CH}$) ; 1733, 1705 et 1699 ($\nu_{C=O}$) ; 1403 et 1368 ($\nu_{CH3}$ *t*-Bu) ; 1248 ($\nu_{C\text{-}C\text{-}O\ asym}$) ; 1163 ($\nu_{O\text{-}C\text{-}C\ asym}$) ; 929 ($\nu_{CH}$ vinyl).
**RMN $^1$H ($CDCl_3$ ; 300 MHz) :** 1,21-1,38 (4H, m, 2$C\underline{H}_2$ cHex) ; 1,44 et 1,47 (9H, s, $C(C\underline{H}_3)_3$ Boc 1er et 2ème conf.) ; 1,61-1,91 (6H, m, 3$C\underline{H}_2$ cHex) ; 2,14-2,43 (3H, m, $C\underline{H}_{2\beta}$ et C$\underline{H}$CO cHex) ; 3,63 (2H, m, $C\underline{H}_2$N) ; 4,35 et 4,45 (1 H, 2t, J = 8,0 Hz et J = 7,7 Hz, $C\underline{H}_\alpha$ 1er et 2ème conf.) ; 4,66 (2H, m, $C\underline{H}_2$-CH=$CH_2$) ; 5,23-5,38 (3H, m, $CH_2$-CH=$C\underline{H}_2$ et C$\underline{H}$-O Hyp) ; 5,92 (1 H, m, $CH_2$-C$\underline{H}$=$CH_2$).

**1.3) Synthèse de la $N^{\alpha}$-Boc-*trans*-4-Hyp(COR)-OH**

Le clivage de chacun des deux esters allyliques **75a** et **75b** selon le mode opératoire de Carrasco et Brown ([31]) conduit respectivement à l'acide **76a** avec un rendement quantitatif et à l'acide **76b** avec un rendement de 95 %.

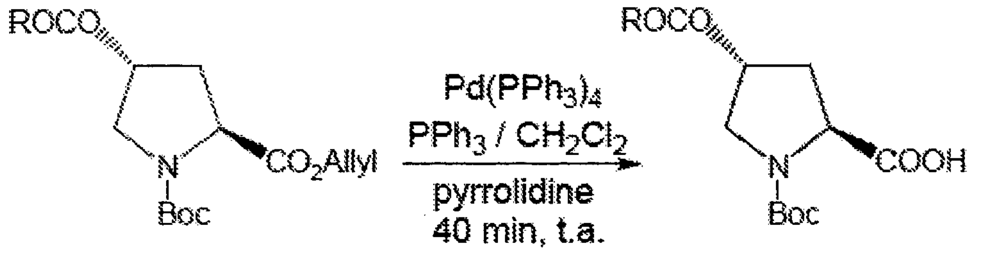


**75a** R = Bn **76a** R = Bn **quantitatif**

**75b** R = cHex **76b** R = cHex **95 %**

**Schéma 3 :** synthèse de la $N^{\alpha}$-Boc-*trans*-4-Hyp(COR)-OH

**Acide (4*R*)-(benzylcarbonyloxy)-*N*-(*tert*-butyloxycarbonyl)-pyrrolidin-(2*S*)-oïque <u>76a</u>**

À une solution de $N^{\alpha}$-Boc-*trans*-4-Hyp(COBn)-OAllyl **<u>75a</u>** (0,311 mmol ; 121 mg) dans 3 mL de $CH_2Cl_2$, sont ajoutés 4 mg de $PPh_3$ (0,01555 mmol ; 0,05 éq) et 27 μl de pyrrolidine (0,3265 mmol ; 1,05 éq) puis le mélange est agité à température ambiante et sous atmosphère d'argon. 9 mg de Pd(0) (0,007775 mmol ; 0,025 éq) sont ensuite ajoutés et le mélange est agité pendant 40 minutes. Le solvant est évaporé et le résidu obtenu est dissous dans l'AcOEt. Cette phase organique est lavée trois fois avec une solution de $KHSO_4$ 0,1 M et une fois avec une solution saturée de NaCl, puis séchée sur du $Na_2SO_4$ anhydre, filtrée et concentrée. Le résidu huileux jaune obtenu est purifié par chromatographie sur colonne de gel de silice (AcOEt / EP 50 : 50 puis avec le même éluant + 0,1 % d'acide formique). Le produit (0,309 mmol ; 108 mg) est obtenu sous la forme d'une huile jaunâtre.
**<u>IR (NaCl ; film)</u> :** 2980 ($\nu_{COOH}$) ; 1732 ($\nu_{C=O}$) ; 1455 ($\nu_{\varphi C=C}$) ; 1369 ($\delta_{CH3}$ t-Bu) ; 1257 ($\nu_{C\text{-}C\text{-}O\ asym}$) ; 1160 ($\nu_{O\text{-}C\text{-}C\ asym}$).
**<u>RMN $^1$H ($CDCl_3$ ; 300 MHz)</u>** : 1,46 et 1,48 (9H, 2s, C($C\underline{H}_3$)$_3$ Boc 1$^{er}$ et 2$^{ème}$ conf.) ; 2,40 (2H, m, $C\underline{H}_{2\beta}$) ; 3,60-3,72 (4H, m+s, $C\underline{H}_2$N et $C\underline{H}_2$Ph) ; 4,32 et 4,44 (1 H, 2t, J = 7,9 Hz et J = 7,9 Hz, $C\underline{H}_{\alpha}$ 1$^{er}$ et 2$^{ème}$ conf.) ; 5,29 (1 H, m, C<u>H</u>OCOBn) ; 7,32 (5H, m, Ph).

**Acide (4*R*)-(cyclohexylcarbonyloxy)-*N*-(*tert*-butyloxycarbonyl)-pyrrolidin-(2*S*)-oïque <u>76b</u>**

Ce produit est préparé selon le même mode opératoire que pour le produit **<u>76a</u>** à partir de la $N^{\alpha}$-Boc-*trans*-4-Hyp(COcHex)-OAllyl **<u>75b</u>** (0,753 mmol ; 287 mg). Le résidu huileux jaune obtenu est purifié par chromatographie sur colonne de gel de silice (AcOEt / EP 50 : 50 puis avec le même éluant + 0,1 % d'acide formique). Le produit (0,718 mmol ; 245 mg) est obtenu sous la forme d'une huile jaunâtre.
**<u>IR (NaCl ; film)</u> :** 3446 ($\nu_{COOH}$) ; 2978 et 2933 ($\nu_{CH}$) ; 1732 ($\nu_{C=O}$) ; 1368 ($\delta_{CH3}$ *t*-Bu) ; 1249 ($\nu_{C\text{-}C\text{-}O\ asym}$).
**<u>RMN $^1$H ($CDCl_3$ ; 300 MHz)</u>** : 1,25-1,41 (4H, m, 2$C\underline{H}_2$ cHex) ; 1,45 et 1,49 (9H, s, C($C\underline{H}_3$)$_3$ Boc 1$^{er}$ et 2$^{ème}$ conf.) ; 1,63-1,90 (6H, m, 3$C\underline{H}_2$ cHex) ; 2,23-2,52 (3H, m, $C\underline{H}_{2\beta}$ et C<u>H</u>CO cHex) ; 3,65 (2H, m, $C\underline{H}_2$N) ; 4,36 et 4,50 (1 H, 2t, J = 8,0 Hz et J = 7,9 Hz, $C\underline{H}_{\alpha}$ 1$^{er}$ et 2$^{ème}$ conf.) ; 5,29 (1 H, d, J = 15,4 Hz, C<u>H</u>-O Hyp) ; 6,30-7,80 (1 H, M, COO<u>H</u>).

**1.4) Synthèse du $N^{\alpha}$-Boc-*trans*-4-Hyp(COR)-Lys(Z)-O*t*Bu**

Les deux acides **76a** et **76b** sont couplés en deux étapes « one-pot » avec la H-Lys(Z)-O*t*Bu.HCl commerciale (Novabiochem Réf. 04-12-5122) selon la méthode de Lai et al. ([25]).

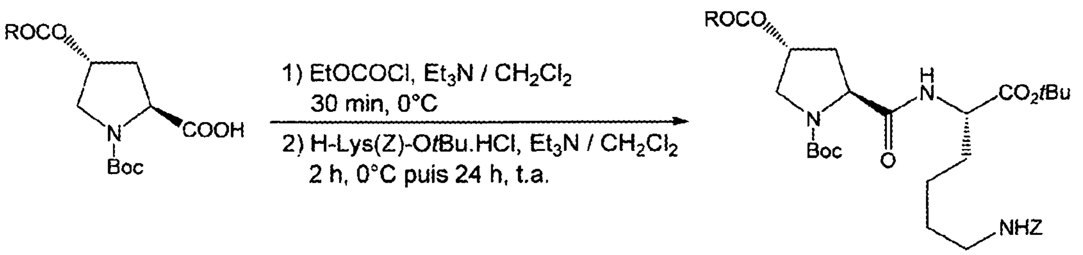


**76a** R = Bn **No. 2** R = Bn **65 %**

**76b** R = cHex **No. 3** = cHex **69 %**

## Schéma 4 : synthèse du $N^{\alpha}$-Boc-*trans*-4-Hyp(COR)-Lys(Z)-O*t*Bu

Le rendement global de chacun des deux dérivés contraints composés **No. 2** et **No. 3** est de 27 % et 53 % respectivement en quatre étapes à partir de la *trans*-4-hydroxy-L-proline commerciale (Novabiochem Réf. 04-10-0020).

***N*$^{\alpha}$-Boc-*trans*-L-4-Hyp(COBn)-L-Lys(Z)-O*t*Bu <u>77a</u>**

La $N^{\alpha}$-Boc-*trans*-4-Hyp(COBn)-OH **<u>76a</u>** (0,30 mmol ; 102 mg) est dissoute dans 4 mL de $CH_2Cl_2$ sous argon. La solution est refroidie à 0°C puis 50 μL de triéthylamine (0,33 mmol ; 1,1 éq) sont ajoutés goutte à goutte pendant 5 minutes. 40 μL de chloroformiate d'éthyle (0,33 mmol ; 1,1 éq) sont ajoutés goutte à goutte pendant 5 minutes. Le mélange est agité à 0°C pendant 30 minutes, puis une solution de H-Lys(Z)-O*t*Bu.HCl commerciale (0,30 mmol ; 112 mg) et de 100 μL de triéthylamine (0,66 mmol ; 2,2 éq) dans 4 mL de $CH_2Cl_2$ à 0°C est ajoutée pendant 5 minutes. La solution est agitée à 0°C pendant deux heures puis à température ambiante pendant la nuit. 5 mL de $CH_2Cl_2$ sont ajoutés et la phase organique est lavée deux fois avec 10 mL d'une solution saturée de $NaHCO_3$ et deux fois avec une solution d'HCl 1M. La phase organique est séchée sur du $Na_2SO_4$ anhydre, filtrée et concentrée. Le résidu huileux jaune obtenu est purifié par chromatographie sur colonne de gel de silice (AcOEt / EP 50 : 50). Le produit (0,195 mmol ; 130 mg) est obtenu sous la forme d'une huile incolore.
**Analyse élémentaire :** trouvé C, 64,93% ; H, 7,37% ; N, 6,06%. $C_{36}H_{49}N_3O_9$ requiert C, 64,75% ; H, 7,40% ; N, 6,29%.
**<u>IR (NaCl ; film)</u> :** 3329 ($\nu_{NH}$) ; 3065 et 3033 ($\nu_{\varphi CH}$) 2978 et 2933 ($\nu_{CH}$) ; 1695 ($\nu_{C=O}$ amide) ; 1455 ($\nu\varphi_{C=C}$) ; 1394 et 1368 ($\delta_{CH3}$ *t*-Bu) ; 1251 ($\nu_{C\text{-}C\text{-}O\ asym}$) ; 1158 ($\nu_{O\text{-}C\text{-}C\ asym}$).
**<u>RMN $^{1}$H ($CDCl_3$ ; 300 MHz)</u> :** 1,44 et 1,47 (9H, 2s, C(C<u>H</u>$_3$)$_3$ Boc 1$^{er}$ et 2$^{ème}$ conf.) ; 1,34-1,69 (6H, m, 3C<u>H</u>$_2$ Lys) ; 2,20 (1 H, m, C<u>H</u>$_\beta$) ; 2,45 (1 H, m, C<u>H</u>$_\beta$) ; 3,17 (2H, m, C<u>H</u>$_2$NH) ; 3,55-3,63 (4H, m+s, C<u>H</u>$_2$N et C<u>H</u>$_2$Ph) ; 4,28 (1 H, m, C<u>H</u>$_\alpha$) ; 4,44 (1 H, m, C<u>H</u>$_\alpha$) ; 5,10 (2H, s, C<u>H</u>$_2$Ph Z) ; 5,25 (1 H, m, C<u>H</u>-O Hyp) ; 7,25-7,37 (10H, m, 2Ph).
**<u>RMN $^{13}$C ($CDCl_3$ ; 75 MHz)</u> :** 22,62 (<u>C</u>H$_2$ Lys) ; 28,38 et 28,64 (6<u>C</u>H$_3$ Boc et t-Bu) ; 29,44 et 30,11 (2<u>C</u>H$_2$ Lys) ; 32,38 (<u>C</u>H$_{2\beta}$) ; 40,96 et 41,72 (<u>C</u>H$_2$NH et <u>C</u>H$_2$Ph Hyp) ; 52,91 (<u>C</u>H$_2$N Hyp) ; 58,97 (<u>C</u>Hα) ; 60,82 (<u>C</u>H$_\alpha$) ; 66,94 (<u>C</u>H$_2$Ph Z) ; 73,68 (<u>C</u>HO Hyp) ; 81,16 (<u>C</u>-O *t*-Bu) ; 82,47 (<u>C</u>-O *t*-Bu) ; 127,66, 128,89, 129,07 et 130,0 (10<u>C</u>H 2Ph) ; 133,94 (<u>C</u>q Ph) ; 137,07 (Cq Ph) ; 155,50 et 156,89 (2CO Boc et Z) ; 171,23, 171,37 et 171,59 (<u>C</u>O amide et 2<u>C</u>O ester).
**<u>SM (ESI ; mode positif)</u>** : 1357,1 [2M+Na]$^+$ ; 690,2 [M+Na]$^+$.
**<u>SM HR (ESI ; mode positif)</u>** : 690,33599 [M+Na]$^+$ (calc. 690,3367).

***N*$^{\alpha}$-Boc-*trans*-L-4-Hyp(COcHex)-L-Lys(Z)-O*t*Bu <u>77b</u>**

Ce dipeptide est préparé selon le même mode opératoire que pour le produit **<u>77a</u>** à partir de la $N^{\alpha}$-Boc-*trans*-4-Hyp(COcHex)-OH **<u>76b</u>** (0,642 mmol ; 219 mg) et de la H-Lys(Z)-O*t*Bu.HCl commerciale (0,642 mmol ; 240 mg). Le résidu pâteux jaunâtre obtenu est purifié par chromatographie sur colonne de gel de silice (AcOEt / EP 40 : 60). Le produit (0,443 mmol ; 292 mg) est obtenu sous la forme d'une huile jaune visqueuse.
**Analyse élémentaire :** trouvé C, 63,95% ; H, 8,20% ; N, 6,30%. $C_{35}H_{53}N_3O_9$ requiert C, 63,71% ; H, 8,10% ; N, 6,37%.
**<u>IR (NaCl ; film)</u> :** 3350 ($\nu_{NH}$) ; 2933 ($\nu_{CH}$) ; 1668 ($\nu_{C=O}$ amide) ; 1454 ($\nu_{\varphi C=C}$) ; 1394 ($\delta_{CH3}$ *t*-Bu) ; 1248 ($\nu_{C\text{-}C\text{-}O\ asym}$) ; 1160 ($\nu_{O\text{-}C\text{-}C\ asym}$).
**<u>RMN $^{1}$H ($CDCl_3$ ; 300 MHz)</u> :** 1,44 et 1,47 (9H, 2s, C(C<u>H</u>$_3$)$_3$ Boc 1$^{er}$ et 2$^{ème}$ conf.) ; 1,23-1,88 (16H, m, 3C<u>H</u>$_2$ Lys et 5C<u>H</u>$_2$ cHex) ; 2,18-2,54 (3H, m, C<u>H</u>$_{2\beta}$ et C<u>H</u>CO cHex) ; 3,19 (2H, m, C<u>H</u>$_2$NH) ; 3,56 (2H, M, C<u>H</u>$_2$N) ; 4,36 (1 H, m, C<u>H</u>$_\alpha$) ; 4,45 (1 H, m, C<u>H</u>$_\alpha$) ; 5,10 (2H, système AB, J = 12,4 Hz, C<u>H</u>$_2$Ph) ; 5,22 (1 H, M, C<u>H</u>-O Hyp) ; 7,05 (1 H, M, N<u>H</u>) ; 7,34 (5H, m, Ph).
**<u>RMN $^{13}$C ($CDCl_3$ ; 75 MHz)</u>** : 21,46, 22,19, 22,64, 29,43, 30,08, 32,35, 32,94 et 34,68 (5<u>C</u>H$_2$ cHex et 3<u>C</u>H$_2$ Lys) ; 28,37 et 28,64 (6<u>C</u>H$_3$ Boc et *t*-Bu) ; 37,32 (<u>C</u>H$_{2\beta}$) ; 43,35 (<u>C</u>HCO cHex) ; 53,11 (<u>C</u>H$_2$NH) ; 59,04 (<u>C</u>H$_\alpha$) ; 60,41 (<u>C</u>H$_\alpha$) ; 60,80 (CH$_2$N) ; 72,81 (<u>C</u>H-O Hyp) ; 73,67 (<u>C</u>H$_2$Ph) ; 81,11 (<u>C</u>-O *t*-Bu) ; 82,43 (<u>C</u>-O *t*-Bu); 128,16, 128,43, 128,51 et 128,87 (5<u>C</u>H Ph) ; 137,06 (<u>C</u>q Ph) ; 155,67 et 156,89 (2CO Boc et Z) ; 171,30, 171,95 et 175,87 (CO amide et 2CO ester).
**<u>SM (ESI ; mode positif)</u> :** 1341,2 [2M+Na]$^+$ ; 682,2 [M+Na]$^+$ ; 660,0 [MH]$^+$. **<u>SM HR (ESI ; mode positif)</u> :** 682,36780 [M+Na]$^+$ (calc. 682,3680).

**2) Synthèse du *N*$^{\alpha}$-Boc-*trans*-4-Hyp(Bn)-Lys(Z)-O*t*Bu**

Cette synthèse se fait par couplage peptidique des deux dérivés commerciaux, la $N^{\alpha}$-Boc-*trans*-4-Hyp(Bn)-OH (Bachem Réf. 54631-81-1) et la H-Lys(Z)-O*t*Bu.HCl (Novabiochem Réf. 04-12-5122) en deux étapes « one pot » et dans les conditions utilisées précédemment ([25]) pour ce type de couplage. Le rendement du dipeptide **No. 4** est de 70 %.

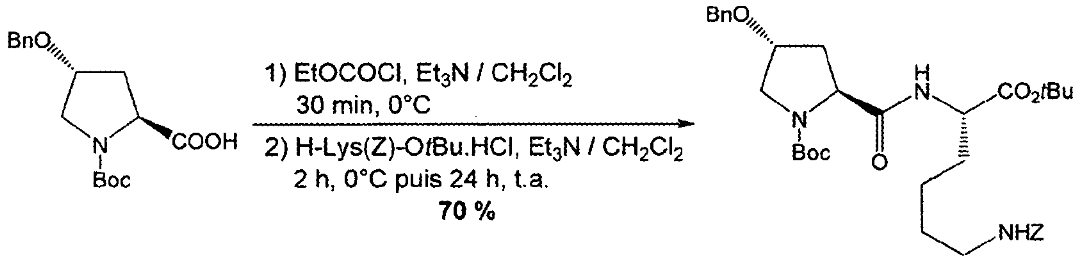


**Composé No. 4**

**Schéma 5 :** synthèse du *N*$^{\alpha}$-Boc-*trans*-4-Hyp(Bn)-Lys(Z)-O*t*Bu

***N*$^{\alpha}$-Boc-*trans*-L-4-Hyp(Bn)-L-Lys(Z)-O*t*Bu <u>78</u>**

Ce dipeptide est préparé selon le même mode opératoire que pour le produit **<u>77a</u>** à partir de la ***N*$^{\alpha}$-Boc-*trans*-4-Hyp(Bn)-OH** commerciale (0,623 mmol ; 200 mg) et de la H-Lys(Z)-O*t*Bu.HCl commerciale (0,623 mmol ; 232 mg). Le résidu huileux jaune obtenu est purifié par chromatographie sur colonne de gel de silice (AcOEt / EP 40 : 60). Le produit (0,438 mmol ; 280 mg) est obtenu sous la forme d'une huile plus ou moins incolore.
**Analyse élémentaire :** trouvé C, 65,77% ; H, 7,66%; N, 6,50%. $C_{35}H_{49}N_3O_8$ requiert C, 65,71% ; H, 7,72% ; N, 6,57%. **<u>IR (NaCl ; film) :</u>** 3325 ($\nu_{NH}$) ; 3065 et 3033 ($\nu_{\varphi CH}$) ; 2977 et 2934 ($\nu_{CH}$) ; 1679 ($\nu_{C=O}$ amide) ; 1455 ($\nu_{\varphi C=C}$) ; 1394 et 1367 ($\nu_{CH3}$ *t*-Bu) ; 1251 ($\nu_{C\text{-}C\text{-}O\ asym}$) ; 1160 ($\nu_{O\text{-}C\text{-}C\ asym}$).
**<u>RMN $^1$H (CDCl$_3$ ; 300 MHz) :</u>** 1,35 et 1,38 (9H, 2s, C(C<u>H</u>$_3$)$_3$ Boc 1$^{er}$ et 2$^{ème}$ conf.) ; 1,23-1,78 (6H, m, 3C<u>H</u>$_2$ Lys) ; 2,07 (1 H, m, C<u>H</u>$_{\beta}$) ; 2,32 (1 H, m, C<u>H</u>$_{\beta}$) ; 3,09 (2H, m, C<u>H</u>$_2$NH) ; 3,44 (2H, M, C<u>H</u>$_2$N) ; 4,24-4,37 (2H, m, 2C<u>H</u>$_{\alpha}$) ; 4,41 (2H, d, J = 3,7 Hz, C<u>H</u>$_2$Ph Hyp) ; 4,96-5,05 (3H, m, C<u>H</u>-O Hyp et C<u>H</u>$_2$Ph Z) ; 6,52 (1 H, M, N<u>H</u>) ; 6,97 (1 H, M, N<u>H</u>) ; 7,20-7,27 (10H, m, 2Ph).
**<u>RMN $^{13}$C (CDCl$_3$ ; 75 MHz) :</u>** 22,67 (<u>C</u>H$_2$ Lys) ; 28,39 et 28,70 (6<u>C</u>H$_3$ Boc et *t*-Bu) ; 29,45 et 32,37 (2<u>C</u>H$_2$ Lys) ; 34,72 (<u>C</u>H$_{2\beta}$) ; 40,97 (<u>C</u>H$_2$NH) ; 52,39 (<u>C</u>H$_2$N) ; 59,24 (<u>C</u>H$_{\alpha}$) ; 60,42 (<u>C</u>H$_{\alpha}$) ; 66,89 (<u>C</u>H$_2$Ph Hyp) ; 71,64 (<u>C</u>H$_2$Ph Z) ; 77,33 (<u>C</u>H-O Hyp) ; 80,84 (<u>C</u>-O *t*-Bu) ; 82,34 (<u>C</u>-O *t*-Bu) ; 128,03, 128,18, 128,50 et 128,87 (10<u>C</u>H 2Ph) ; 137,09 (<u>C</u>q Ph) ; 138,28 (<u>C</u>q Ph) ; 155,76 et 156,89 (2CO Boc et Z) ; 171,62 et 172,72 (<u>C</u>O amide et <u>C</u>O ester). **<u>SM (ESI ; mode positif)</u>** : 662,3 [M+Na]$^+$ ; 540,2 [MH]$^+$- isobutène - $CO_2$. **<u>SM HR (ESI ; mode positif)</u>** : 662,34155 [M+Na]$^+$ (calc. 662,3417).

**3) Synthèse du *N*$^{\alpha}$-Boc-*trans*-4-Hyp(CH$_2$cHex)-Lys(Z)-O*t*Bu**

La stratégie de synthèse met une jeu une réaction de couplage peptidique entre la *N*$^{\alpha}$-Boc-*trans*-4-Hyp (CH$_2$cHex)-OH et la H-Lys(Z)-O*t*Bu.HCl commerciale (Novabiochem Réf. 04-12-5122). Le passage à l'éther cyclohexylméthylénique de la *N*$^{\alpha}$-Boc-*trans*-4-Hyp-OH peut être réalisé par hydrogénation catalytique de l'éther benzylique de la *N*$^{\alpha}$-Boc-*trans*-4-Hyp(Bn)-OH commerciale (Bachem Réf. 54631-81-1).

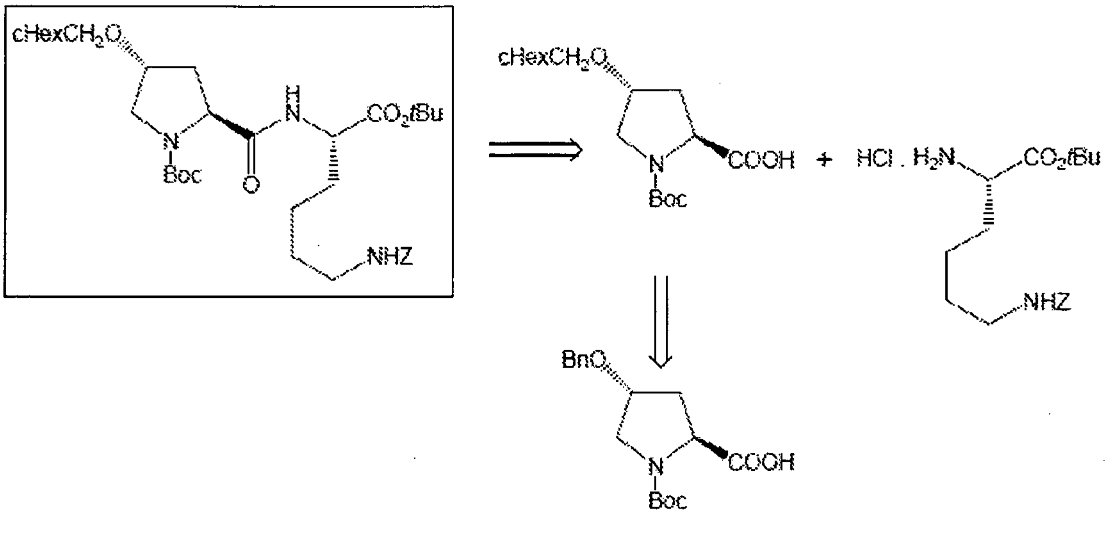

**3.1) Synthèse de la *N*α-Boc-*trans*-4-Hyp($CH_2$cHex)-OH**

Seko et coll. (SEKO T., KATO M., KOHNO H., ONO S., HASHIMURA K., TAKIMIZU H., NAKAI K., MAEGAWA H., KATSUBE N., TODA M. Bioorg. Med. Chem., 2003, 11(8), 1901-1913, [28]) ont décrit la synthèse de la *N*α-Boc-L-Ser($CH_2$cHex)-OH par hydrogénation catalytique de la *N*α-Boc-L-Ser(Bn)-OH en présence de rhodium sur alumine (Rh-$Al_2O_3$) dans l'isopropanol. L'éther cyclohexylméthylénique de la sérine *N*α-protégée est obtenu avec un rendement de 86 % après recristallisation dans l'hexane.

La réaction est réalisée dans les mêmes conditions sur la *N*α-Boc-*trans*-4-Hyp(Bn)-OH commerciale (Bachem Réf. 54631-81-1) et l'éther cyclohexylméthylénique **79** est obtenu avec un rendement quantitatif.

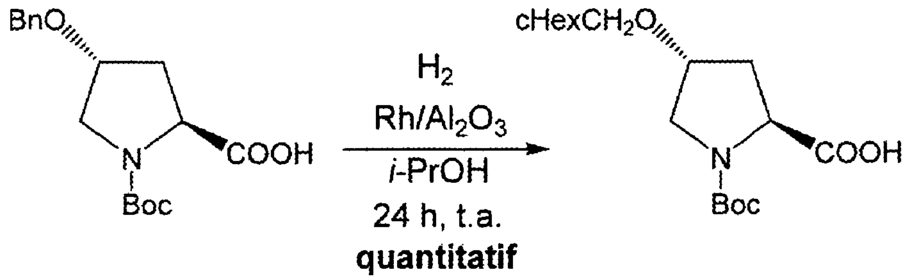


**79**

**Schéma 6 :** synthèse de la *N*α-Boc-*trans*-4-Hyp(CH2cHex)-OH

**Acide (4*R*)-(cyclohexylméthyloxy)-*N*-(*tert*-butyloxycarbonyl)-pyrrolidin-(2*S*)-oïque 79**

Un mélange de *N*α-Boc-*trans*-4-Hyp(Bn)-OH commercial (0,623 mmol ; 200 mg) et de Rh/$Al_2O_3$ (5 %) (0,018 mmol ; 37 mg ; 0,028 éq) dans 4 mL d'isopropanol, est agité à température ambiante et sous atmosphère d'hydrogène pendant la nuit. Le mélange est filtré afin d'éliminer le catalyseur et le filtrat est concentré. Le produit (0,62 mmol ; 203 mg) est obtenu sous la forme d'une huile plus ou moins incolore qui ne nécessite pas de purification.

**IR (NaCl ; film)** : 3418 ($\nu_{COOH}$) ; 2974 et 2924 ($\nu_{CH}$) ; 1705 et 1682 ($\nu_{C=O}$) ; 1368 ($\delta_{CH3}$ *t*-Bu) ; 1257 ($\nu_{C\text{-}C\text{-}O\ asym}$) ; 1163 ($\nu_{O\text{-}C\text{-}C\ asym}$).

**RMN $^1$H ($CDCl_3$ 300 MHz) :** 1,17-1,27 (6H, m, 3C$H_2$ cHex) ; 1,43 et 1,50 (9H, s, C(C$H_3$)$_3$ Boc 1er et 2éme conf.) ; 1,65-1,76 (5H, m, 2C$H_2$ et CH cHex) ; 2,16 (1 H, m, C$H_\beta$) ; 2,39 (1 H, m, C$H_\beta$) ; 3,21 (2H, m, C$H_2$N) ; 3,54 (2H, m, C$H_2$O) ; 4,05 (1 H, m, CH-O) ; 4,34 et 4,46 (1 H, 2t, J = 7,7 Hz et J = 7,3 Hz, C$H_\alpha$) ; 5-5,90 (1 H, M, COOH).

**ii - Synthèse du *N*α-Boc-*trans*-4-Hyp($CH_2$cHex)-Lys(Z)-O*t*Bu**

L'acide 79 est couplé avec la H-Lys(Z)-O*t*Bu.HCl en deux étapes « one-pot » dans les conditions de synthèse du composé 77a. Ce couplage a abouti au dipeptide 80 avec un rendement de 67%.

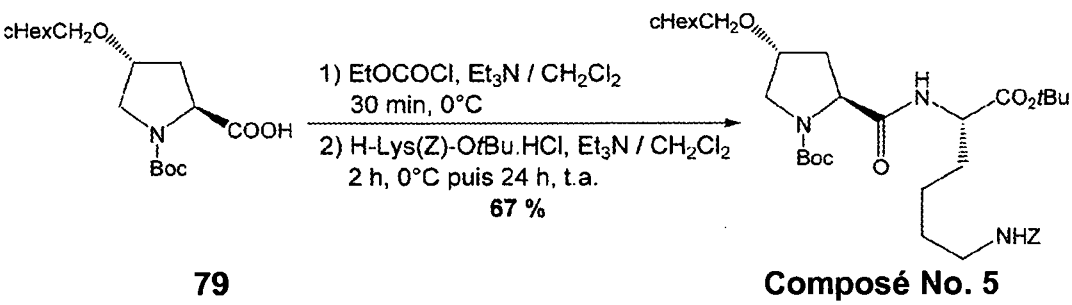


**79** **Composé No. 5**

**Schéma 7 :** obtention du *N*α-Boc-*trans*-4-Hyp(CH2cHex)-Lys(Z)-O*t*Bu

***N*α-Boc-*trans*-L-4-Hyp($CH_2$cHex)-L-Lys(Z)-O*t*Bu 80**

Ce dipeptide est préparé selon le même mode opératoire que pour le produit **77a** à partir de la *N*α-Boc-*trans*-4-Hyp($CH_2$cHex)-OH **79** (0,62 mmol ; 203 mg) et de la H-Lys(Z)-O*t*Bu.HCl commerciale (0,62 mmol ; 231 mg). Le résidu huileux jaunâtre obtenu est purifié par chromatographie sur colonne de gel de silice (AcOEt / EP 40 : 60). Le produit

(0,414 mmol ; 267 mg) est obtenu sous la forme d'une huile jaune.

**Analyse élémentaire :** trouvé C, 65,34% ; H, 8,78% ; N, 6,40%. $C_{35}H_{55}N_3O_8$ requiert C, 65,09% ; H, 8,58% ; N, 6,51 %.

**IR (NaCl ; film) :** 3325 ($\nu_{NH}$) ; 3066 et 3034 ($\nu_{\varphi CH}$) ; 2977 et 2925 ($\nu_{CH}$) ; 1681 ($\nu_{C=O}$ amide) ; 1454 ($\nu_{\varphi C=C}$) ; 1394 et 1368 ($\delta_{CH3}$ *t*-Bu) ; 1248 (νc-c-o $_{asym}$) ; 1159 ($\nu_{O\text{-}C\text{-}C\ asym}$).

**RMN $^1$H ($CDCl_3$ ; 300 MHz) :** 1,44 et 1,47 (9H, 2s, $C(C\underline{H}_3)_3$ Boc 1$^{er}$ et 2$^{ème}$ conf.) ; 1,12-1,89 (17H, m, 3C$\underline{H}_2$ Lys, 5C$\underline{H}_2$ et C$\underline{H}$ cHex) ; 2,06 (1H, m, C$\underline{H}_\beta$) 2,34 (1H, m, C$\underline{H}_\beta$) ; 3,13-3,21 (4H, m, 2C$\underline{H}_2$N) ; 3,46 (2H, M, C$\underline{H}_2$O Hyp) ; 3,95 et 4,04 (1 H, 2M, C$\underline{H}_\alpha$ 1$^{er}$ et 2$^{ème}$ conf.) ; 4,31 (1 H, m, C$\underline{H}_\alpha$) ; 4,43 (1 H, m, C$\underline{H}$-O Hyp) ; 5,10 (3H, M+s, N$\underline{H}$ et C$\underline{H}_2$Ph) ; 7,06 (1 H, d, J = 7,9 Hz, N$\underline{H}$) ; 7,36 (5H, s, Ph).

**RMN $^{13}$C ($CDCl_3$ ; 75 MHz) :** 21,43, 22,30, 22,68, 29,43, 30,08, 30,37, 30,43 et 32,39 (5$\underline{C}H_2$ cHex et 3$\underline{C}H_2$ Lys) ; 28,38 et 28,68 (6$\underline{C}H_3$ Boc et t-Bu) ; 34,73 ($\underline{C}H_{2\beta}$) ; 38,46 ($\underline{C}$H cHex) ; 41,0 ($\underline{C}H_2$NH) ; 52,33 ($\underline{C}H_2$N) ; 52,92 ($\underline{C}H_\alpha$) ; 59,25 ($\underline{C}H_\alpha$) ; 66,90 ($\underline{C}H_2$O Hyp) ; 75,48 ($\underline{C}H_2$Ph) ; 77,59 ($\underline{C}$H-O Hyp) ; 80,71 ($\underline{C}$-O *t*-Bu) ; 82,29 ($\underline{C}$-O *t*-Bu) ; 128,46 et 128,86 (5$\underline{C}$H Ph) ; 137,10 ($\underline{C}$q Ph) ; 155,89 et 156,86 (2CO Boc et Z) ; 171,61 et 172,87 ($\underline{C}$O amide et $\underline{C}$O ester).

**SM (ESI ; mode positif)** : 1313,3 $[2M+Na]^+$ ; 668,3 $[M+Na]^+$ ; 646,1 $[MH]^+$. **SM HR (ESI ; mode positif)** : 668,38834 $[M+Na]^+$ (calc. 668,3887).

#### 4) Synthèse du *N*$^\alpha$-Boc-*trans*-4-Hyp(Bn)-ψ($CH_2$-NH)-Lys(Z)-O*t*Bu

L'analogue aminométhylénique du composé No. 4 est synthétisé par une réaction d'amination réductrice entre l'aldéhyde dérivé de la *trans-4*-hydroxy-L-proline doublement protégée et la lysine protégée selon le schéma rétrosynthétique représentée ci-dessous.

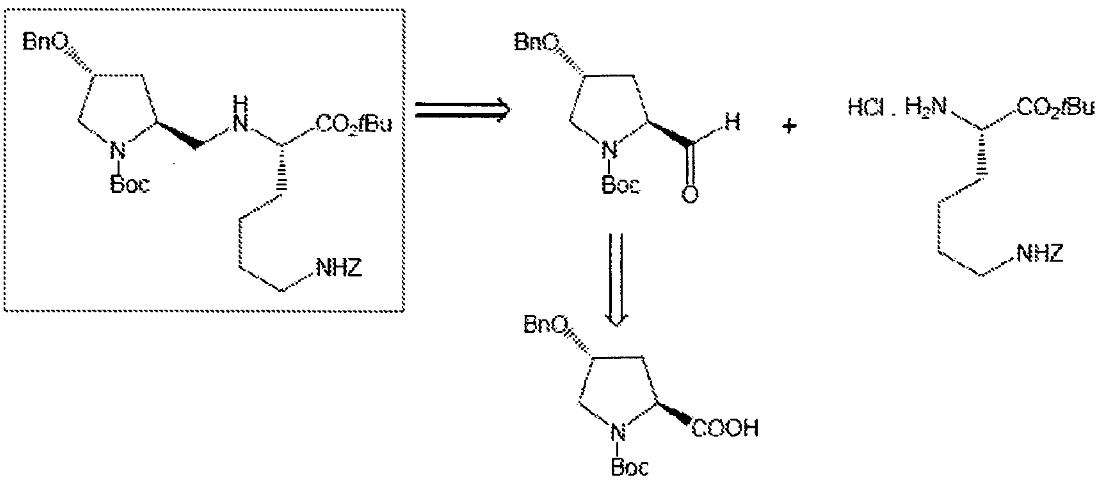

La lysine protégée utilisée dans cette réaction est commerciale (Novabiochem Réf. 04-12-5122). La synthèse de l'aldéhyde est réalisée par réduction de l'amide de Weinreb dérivé de la *trans*-4-hydroxy-L-proline doublement protégée commerciale (Novabiochem Réf. 04-10-0020) selon le mode opératoire de Fehrentz et Castro (FEHRENTZ J.-A., CASTRO B. Synthesis, 1983, 676-678, [29]).

#### 4.1) Synthèse de la *N*$^\alpha$-Boc-*trans*-4-Hyp(Bn)-N(OMe)Me

Le mode opératoire de Fehrentz et Castro [29]) est appliqué pour la synthèse de l'amide de Weinreb **81** à partir de la *N*$^\alpha$-Boc-*trans*-4-Hyp(Bn)-OH commerciale (Novabiochem Réf. 04-10-0020) avec un rendement de 66 %. Le BOP (hexafluorophosphate de benzotriazol-1-yloxy-tris(diméthylamino) phosphonium) utilisé comme agent de couplage est remplacé par le TBTU (tétrafluoroborate de 2-(1-hydroxy-benzotriazol-1-yl)-1,1,3,3-Tétrahydrouronium).

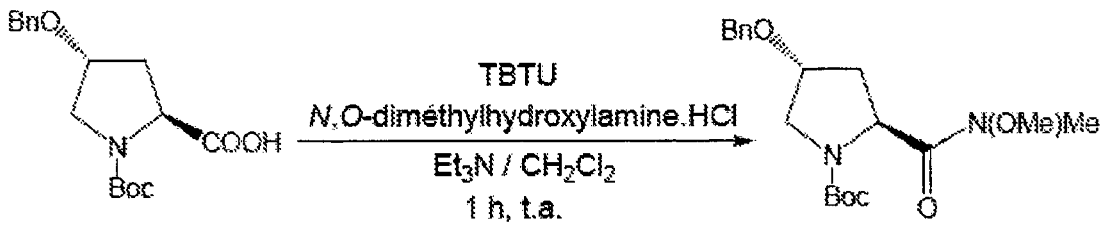


**81**

**Schéma 8 :** synthèse de la *N*$^{\alpha}$-Boc-*trans*-4-Hyp(Bn)-N(OMe)Me

**(4*R*)-(Benzyloxy)-*N*-(*tert*-butyloxycarbonyl)-*N*-méthoxy-*N*-méthyl-pyrrolidin-(2S)-amide 81**

À une solution de *N*$^{\alpha}$-Boc-*trans*-4-Hyp(Bn)-OH commercial (1,83 mmol ; 586 mg) dans 10 mL de $CH_2Cl_2$, sont ajoutés 257 μL de triéthylamine (1,83 mmol ; 1 éq) suivis de 586 mg de TBTU (1,83 mmol ; 1éq). Après 5 minutes d'agitation à température ambiante, 214 mg du chlorhydrate de la N,O-diméthylhydroxylamine (2,19 mmol ; 1,2 éq) sont ajoutés et à nouveau 309 μL de triéthylamine (2,2 mmol ; 1,2 éq). La réaction est agitée à température ambiante pendant 24 heures. 10 mL de $CH_2Cl_2$ sont ajoutés et la réaction est lavée trois fois avec chacune des solutions suivantes : une solution d'HCl à 10 %, une solution saturée de $NaHCO_3$ et une solution saturée de NaCl. La phase organique est séchée sur du $Na_2SO_4$ anhydre filtrée et concentrée. Le résidu huileux plus ou moins incolore obtenu est purifié par chromatographie sur colonne de gel de silice (AcOEt / EP 50 : 50). Le produit (1,063 mmol ; 387 mg) est obtenu sous la forme d'une huile jaune.

**IR (NaCl ; film) :** 3064 et 3031 ($\nu_{\varphi CH}$) ; 2975 et 2935 ($\nu_{CH}$) ; 1736, 1695 et 1683 ($\nu_{C=O}$) ; 1455 ($\nu_{\varphi C=C}$) ; 1403 et 1367 ($\delta_{CH3}$ *t*-Bu) ; 1250 ($\nu_{C\text{-}C\text{-}O\ asym}$) ; 1164 ($\nu_{O\text{-}C\text{-}C\ asym}$).

**RMN $^1$H ($CDCl_3$ ; 300 MHz)** : 1,43 et 1,47 (9H, 2s, $C(C\underline{H}_3)_3$ Boc 1$^{er}$ et 2$^{ème}$ conf.) ; 2,04 (1 H, m, $C\underline{H}_{\beta}$) ; 2,38 (1 H, m, $C\underline{H}_{\beta}$), 3,21 (3H, s, $C\underline{H}_3N$) ; 3,56-3,80 (5H, m+2s, $C\underline{H}_2N$ et $C\underline{H}_3O$ 1$^{er}$ et 2$^{ème}$ conf.) ; 4,19 et 4,26 (1 H, 2m, $C\underline{H}_{\alpha}$ 1$^{er}$ et 2$^{ème}$ conf.) ; 4,53 (2H, m, $C\underline{H}_2Ph$) ; 4,78 et 4,86 (1 H, 2t, J = 7,5 Hz et J = 7,3 Hz, C$\underline{H}$OBn 1$^{er}$ et 2$^{ème}$ conf.) ; 7,34 (5H, m, Ph).

**4.2) Synthèse de la *N*$^{\alpha}$-Boc-*trans*-4-Hyp(Bn)-al**

L'amide de Weinreb 81 est réduit en présence d'hydrure de lithium et d'aluminium dans l'éther éthylique pendant 30 minutes à 0°C selon le mode opératoire de Fehrentz et Castro ([29]). Le rendement de l'aldéhyde 82 ainsi obtenu est de 69 %.

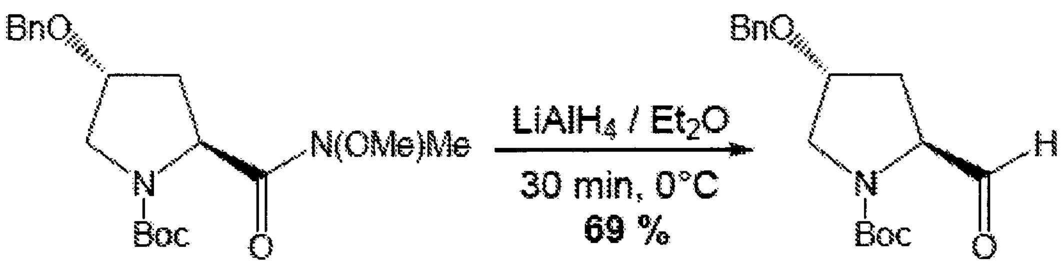


**81** **82**

**Schéma 9 :** synthèse de la *N*$^{\alpha}$-Boc-*trans*-4-Hyp(Bn)-al

**(4R)-(Benzyloxy)-N-(tert-butyloxycarbonyl)-pyrrolidin-(2S)-al 82**

À 50 mg d'hydrure de lithium et d'aluminium (1,25 mmol ; 1,25 éq) refroidi dans un bain de glace, est ajoutée goutte à goutte une solution de *N*$^{\alpha}$-Boc-*trans*-4-Hyp(Bn)-N(OMe)Me 81 (1 mmol ; 360 mg) dans 6 mL d'$Et_2O$ anhydre. Le mélange est laissé sous agitation à 0°C pendant 30 minutes puis hydrolysé avec 5 mL d'une solution de $KHSO_4$ 0,3 M. La phase organique est séparée et la phase aqueuse est extraite trois fois avec de l'$Et_2O$. Les phases éthérées sont rassemblées puis lavées trois fois avec chacune des solutions suivantes : une solution d'HCl à 10 %, une solution saturée de $NaHCO_3$ et une solution saturée de NaCl. La phase organique finale est séchée sur du $Na_2SO_4$ anhydre, filtrée et concentrée. Le résidu huileux jaune obtenu est purifié par chromatographie sur colonne de gel de silice (AcOEt / EP 40 : 60). Le produit (0,688 mmol ; 210 mg) est obtenu sous la forme d'une huile jaunâtre.

**IR (NaCl ; film) :** 3065 et 3032 ($\nu_{\varphi CH}$) 2978 et 2931 ($\nu_{CH}$) ; 2715 ($\nu_{CHO}$) ; 1738, 1704 et 1694 ($\nu_{C=O}$) ; 1455 ($\nu_{\varphi C=C}$) ; 1398 et 1367 ($\delta_{CH3}$ *t*-Bu) ; 1256 ($\nu_{C\text{-}C\text{-}O\ asym}$) ; 1163 ($\nu_{O\text{-}C\text{-}C\ asym}$).
**RMN $^1$H (CDCl$_3$ ; 300 MHz) :** 1,43 et 1,49 (9H, 2s, C(C$\underline{H}_3$)$_3$ Boc 1$^{er}$ et 2$^{ème}$ conf.) ; 1,98 (1 H, m, C$\underline{H}_\beta$) ; 2,28 (1 H, m, C$\underline{H}_\beta$) , 3,55 et 3,81 (2H, 2m, C$\underline{H}_2$N 1$^{er}$ et 2$^{ème}$ conf.) ; 4,15 (1 H, m, C$\underline{H}_\alpha$) ; 4,25 et 4,36 (1 H, 2m, C$\underline{H}$OBn 1$^{er}$ et 2$^{ème}$ conf.) ; 4,54 (2H, système AB, *J* = 11,6 Hz, C$\underline{H}_2$Ph) ; 7,34 (5H, m, Ph) ; 9,45 et 9,57 (1 H, 2d, J = 3,7 Hz et J = 2,6 Hz, C$\underline{H}$O 1$^{er}$ et 2$^{ème}$ conf.).

**4.3) Synthèse du *N*$^\alpha$-Boc-*trans*-4-Hyp(Bn)-ψ(CH$_2$-NH)-Lys(Z)-O*t*Bu**

L'aldéhyde 82 est engagé dans une réaction d'amination réductrice avec un excès (1,2 équivalents) de lysine protégée commerciale (Novabiochem Réf. 04-12-5122), selon le même mode opératoire que celui décrit dans Martinez et al. (MARTINEZ J., BALI J . P., RODRIGUEZ M., CASTRO B., MAGOUS R., LAUR J., LIGNON M-F. J. Med. Chem., 1985, 28(12), 1874-1879, [30]). L'analogue aminométhylénique composé No. 6 est obtenu après une heure de réaction à température ambiante avec un rendement de 52 %.

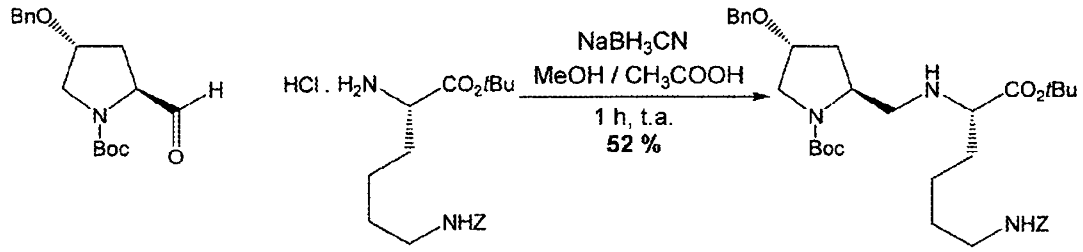


**82** **Composé N° 6**

**Schéma 10 :** obtention du *N*$^\alpha$-Boc-*trans*-4-Hyp(Bn)-ψ(CH$_2$-NH)-Lys(Z)-O*t*Bu

Le dipeptide réduit No. 6 est obtenu avec un rendement global de 24 % en trois étapes à partir de la *N*$^\alpha$-Boc-*trans*-4-Hyp(Bn)-OH commerciale (Novabiochem Réf. 04-10-0020).

***N*$^\alpha$-Boc-*trans*-L-4-Hyp(Bn)-ψ(CH$_2$-NH)-L-Lys(Z)-O*t*Bu 83**

L'aldéhyde **82** (0,68 mmol ; 207 mg) est dissous dans 10 mL de MeOH / $CH_3COOH$ (99 : 1) contenant 300 mg de H-Lys(Z)-O*t*Bu.HCl commerciale (0,816 mmol; 1,2 éq). 34 mg de cyanoborohydrure de sodium (0,544 mmol ; 0,8 éq) sont ajoutés par petites portions pendant 45 minutes et sous agitation. Après agitation pendant une heure à température ambiante, le ballon est plongé dans un bain d'eau glacée, puis 15 mL d'une solution saturée de $NaHCO_3$ sont ajoutés suivis de 20 mL d'AcOEt. La phase organique est séparée, lavée avec 10 mL d'eau puis séchée sur du $Na_2SO_4$ anhydre, filtrée et concentrée. Le résidu huileux obtenu est purifié par chromatographie sur colonne de gel de silice (AcOEt / EP 40 : 60). Le produit (0,350 mmol ; 219 mg) est obtenu sous la forme d'une huile jaunâtre.
**Analyse élémentaire :** trouvé C, 66,91% ; H, 8,53% ; N, 6,77%. $C_{35}H_{51}N_3O_7$ requiert C, 67,17% ; H, 8,21 % ; N, 6,71 %.
**IR (NaCl ; film) :** 3343 ($\nu_{NH}$) ; 3065 et 3033 ($\nu_{\varphi CH}$) ; 2976 et 2933 ($\nu_{CH}$) ; 1728, 1716, 1704, 1694 et 1682 ($\nu_{C=O}$) ; 1455 ($\nu_{\varphi C=C}$) ; 1394 et 1367 ($\delta_{CH3}$ *t*-Bu) ; 1247 ($\nu_{C\text{-}C\text{-}O\ asym}$) ; 1162 ($\nu_{O\text{-}C\text{-}C\ asym}$).
**RMN $^1$H (CDCl$_3$ ; 300 MHz) :** 1,46 (9H, 2s, C(C$\underline{H}_3$)$_3$ Boc) ; 1,35-1,68 (6H, m, 3C$\underline{H}_2$ Lys) ; 2,11 (2H, m, C$\underline{H}_{2\beta}$) ; 2,65 (2H, m, C$\underline{H}_2$NH) ; 3,19 (2H, q, J = 6,4 Hz, C$\underline{H}_2$NHZ) ; 3,45 (2H, m, C$\underline{H}_2$N) ; 3,91-4,14 (2H, m, 2C$\underline{H}_\alpha$) ; 4,49 (2H, système AB, J = 13,3 Hz, C$\underline{H}_2$Ph Hyp) ; 4,80 (1 H, M, C$\underline{H}$OBn) ; 5,10 (2H, s, C$\underline{H}_2$Ph Z) ; 7,31-7,38 (10H, m, 2Ph).
**RMN $^{13}$C (CDCl$_3$ : 75 MHz) :** 23,45 ($\underline{C}$H$_2$ Lys) ; 28,53 et 28,87 (6$\underline{C}$H$_3$ Boc et *t*-Bu) ; 30,07 et 33,65 (2$\underline{C}$H$_2$ Lys) ; 41,27 ($\underline{C}$H$_{2\beta}$) ; 51,13, 51,31 et 51,96 (3$\underline{C}$H$_2$N) ; 56,65 ($\underline{C}$H$_\alpha$) ; 62,66 ($\underline{C}$H$_\alpha$) ; 66,86 ($\underline{C}$H$_2$Ph Hyp) ; 71,25 ($\underline{C}$H$_2$Ph Z) ; 76,60 ($\underline{C}$H-O Hyp) ; 79,96 ($\underline{C}$-O *t*-Bu) ; 81,39 ($\underline{C}$-O *t*-Bu) ; 128,06, 128,51, 128,82 et 128,91 (10$\underline{C}$H 2Ph) ; 137,03 ($\underline{C}$q Ph) ; 138,52 ($\underline{C}$q Ph) ; 155,37 et 156,78 (2$\underline{C}$O Boc et Z) ; 175,19 ($\underline{C}$O ester).
**SM (ESI ; mode positif)** : 626,2 [MH]$^+$ ; 570,2 [MH]$^+$ - isobutène ; 514,1 [MH]$^+$ - 2isobutène ; 470,3 [MH]$^+$ - 2isobutène - $CO_2$.
**SM HR (ESI ; mode positif)** : 626,38092 [MH]$^+$ (calc. 626,3805).

**EXEMPLE 2 : ACTIVITÉ BIOLOGIQUE DES COMPOSÉS No. 2, 3, 4, 5 et 6**

**1) Protocoles**

***1.1) Cultures cellulaires***

La lignée de fibroblastes humains HEK-293 (Collection cellulaire *ATCC* CRL-1573) est transfectée avec soit BCRP (Robey R.W. et al. 2003, Br. J. Cancer 89(10) 1971-1978, [32]) soit ABCC1, clonés dans le vecteur d'expression pcDNA3.1 (Invitrogen), ou avec le vecteur vide pcDNA3.1. La lignée cellulaire NIH3T3 (ATCC CRL-1658) a été transfectée de manière stable par pHaMDR1/A (Cardarelli et al. 1995 Cancer Res 55 1086-1091, [33]), vecteur rétroviral exprimant la protéine sauvage MDR1. La lignée cellulaire BHK-21 (ATCC CCL-10) a été transfectée avec MRP1 cloné dans le vecteur d'expression pcDNA3.1 ou le vecteur vide pcDNA3.1. Les cellules sont maintenues en culture dans du DMEM glutamax II (Invitrogen) supplémenté de 10% de sérum foetal bovin et de 1% de pénicilline/streptomycine. Les données traitées avec Sigmaplot V11 (http://www.systat.com/), et l'équation d'ajustement mathématique des données expérimentales pour le calcul des $IC_{50}$ :

$$f=a*(1-\exp(-b*x)) \text{ (a= pente, b = ordonnée à l'origine).}$$

***1.2) Cytométrie en flux***

Les cellules exprimant P-gp, BCRP et MRP, et leurs contrôles correspondants, sont mises en présence de 10 et 2 $\mu$M de mitoxantrone pour les 2 premiers transporteurs et de 1 $\mu$M de daunorubicine pour le 3$^{ème}$. L'accumulation de substrat est réalisée pendant 30 minutes à 37°C en présence ou en absence de concentrations variées des molécules testées à 0,5% de DMSO (diméthylsulfoxyde) final. Après lavage par du PBS (Phosphate buffered saline), les cellules sont incubées dans du milieu contenant les mêmes concentrations de molécules testées que précédemment, pendant 1h à 37°C. La fluorescence intracellulaire d'anticancéreux mitoxantrone et daunorubicine est mesurée avec le FACscan flow cytometer (Becton Dickinson, Moutain, View, CA). Les drogues sont excitées à 488 nm. L'émission de mitoxantrone est mesurée à 650 nm tandis que l'émission de daunorubicine est mesurée à 530 nm. L'efficacité des molécules testées est estimée par l'équation 1 : % efficacité = 100 x ($F_A$ - $F_{BG}$) / ($F_C$ - $F_{BG}$), où $F_A$ correspond au niveau intracellulaire de substrat mesuré dans les cellules surexprimant ABCG2 (correspondant au plus faible niveau de fluorescence en absence d'inhibiteur), $F_C$ correspond à la fluorescence dans les cellules contrôles transfectées avec le vecteur vide (et donc à l'accumulation maximale de substrat dans les cellules), et $F_{BG}$ correspond à la fluorescence mesurée en absence de substrat et en présence d'inhibiteur.

***1.3) Test de cytotoxicité***

Les cellules sont ensemencées à raison de 10 000 par puits dans des plaques 96 puits et incubées pendant 24 heures à 37°C sous 5% de $CO_2$. Des concentrations variées de composés No. 2, 3, 4, 5 et 6, jusqu'à 20 fois l'$IC_{50}$ sont ensuite additionnées. La cytotoxicité est évaluée colorimétriquement avec le 3-(4,5-dimethylthiazol-2-yl)-2,5-diphenyltetrazolium bromide (MTT).

**2) Résultats**

***2.1) Effet inhibiteur des composés de l'invention de l'activité d'efflux des drogues de la P-gp***

L'effet inhibiteur des composés est quantifié en mesurant par cytométrie en flux l'efflux d'une drogue anticancéreuse transportée par la pompe considérée, ie la mitoxantrone pour la P-gp et BCRP et la daunorubicine pour MRP1. Il est comparé à celui du produit de référence, la réversine 121. Les produits sont testés à 10 $\mu$M, puis à 2 $\mu$M pour ceux qui bloquent au moins 80% de l'efflux de mitoxantrone, et pour certains, une gamme plus complète de concentration est testée de manière à établir la valeur de concentration produisant 50% d'inhibition, $IC_{50}$, comme l'indique la figure 3. La figure 3 illustre en effet les pourcentage d'inhibition de l'efflux de la mitoxantrone en fonction de la concentration ($\mu$M) des composés suivants: réversine 121, composé No. 3, composé No. 4, composé No. 5, composé No. 6. Les valeurs correspondantes sont indiquées dans le tableau 2 ci-dessous.

Tableau 2. Effet inhibiteur des composés de l'invention sur les transporteurs ABC MDR

| # | % Inhibition de la pompe à 10 μM de composé | | | $IC_{50}$ P-gp, μM |
|---|---|---|---|---|
| | P-gp | BCRP | MRP1 | |
| Réversine 121, No. 1 | 75.8±1.6 | 37.2±3.7 | - | 1.41±0.34 |
| No. 2 | 68.0±10.0 | 10.0±7.5 | - | - |
| No. 3 | 105.7±13.4 | 11.2±4.6 | - | 1.68±0.28 |
| No. 4 | 99.7±11.0 | 16.6±3.4 | <10 | 0.73±0.20 |
| No. 5 | 82.2±10.8 | 18.8±10.0 | - | 1.13±0.34 |
| No. 6 | 106.9±23.0 | 42.3±0.8 | <10 | 0.22±0.03 |
| «-» signifie non mesurable | | | | |

Tous les composés sont actifs, et présentent notamment une activité compatible avec leur utilisation pour réduire l'activité d'efflux d'un anti-cancéreux de P-gp.

La plupart des composés sont plus actifs que la réversine 121 à une concentration de 10 μM.

L'$IC_{50}$ pour la P-gp du composé No. 3 est meilleure que celle de la réversine, et est du même ordre de grandeur que celle-ci. Sa capacité d'inhibition de BCRP et MRP1 es faible ou nulle avec 11,2% pour BCRP et non mesurable pour MRP1. L'$IC_{50}$ du composé No. 4 est 2 fois meilleure que celle de la réversine 121, avec toujours une capacité d'inhibition de BCRP et de MRP1 faible ou nulle. L'$IC_{50}$ du composé No. 6, est 7 fois meilleure que celle de la réversine 121.

### *2.2) Sélectivité*

Le tableau 2 montre que les composés sont spécifiques de P-gp, et peu ou pas actifs sur BCRP et MRP1, ce qui permet d'atteindre l'objectif de disposer dans un contexte médical de produits moins toxiques, préservant ainsi le rôle physiologique des transporteurs non impliqués dans la résistance à une chimiothérapie donnée.

### *2.3) Chimiosensibilisation*

La chimiosensibilisation est illustrée ici dans la figure 4, représentant le pourcentage de survie cellulaire de cellules NIH3T3 exprimant (triangles, losanges) ou n'exprimant pas (cercles, carrés) la P-gp, en fonction de la concentration (μM) de mitoxantrone administrée et de la présence (1μM, carrés, losanges) ou de l'absence (cercles, triangles) du composé No. 6.

Une expérience similaire a également été réalisée avec le composé No.4.

En absence des composés No. 4 ou 6, les cellules exprimant la P-gp sont 10 fois plus résistantes que celles ne l'exprimant pas (triangles *vs* cercles). L'addition d'une concentration équivalente à 5 fois l'$IC_{50}$, soit 4 μM pour le composé No. 4 et 1 μM pour le composé No. 6 restaure complètement la sensibilité des cellules surexprimant la P-gp à l'anticancéreux utilisé, la mitoxantrone.

### *2.4) Cytotoxicité*

Des concentrations croissantes des composés No. 4 ou No. 6 sont appliquées à des cellules NIH3T3 exprimant ou n'exprimant pas la P-gp. La survie des cellules est mesurée après 72 h et est exprimée en pourcentage. La cytotoxicité est illustrée dans la figure 5. À 10 fois la concentration de ½ effet maximal, ces deux composés ne sont pas toxiques. Au-delà, la toxicité du composé No. 4 reste négligeable tandis que celle du composé No. 6 reste limitée.

Cette absence de cytotoxicité est donc compatible avec l'administration de ces composés chez un mammifère.

### *2.5) Mode d'inhibition*

La Figure 6 montre clairement que le mécanisme d'inhibition du composé 6 est du type non compétitif, particulièrement visible en représentation en double inverse de Lineweaver & Burke sur le panneau de droite. La concentration intracellulaire de daunorubicine fluorescente est mesurée à des concentrations croissantes de daunorubicine, en présence ou en absence de concentrations fixes de 0, 0,12 μM, 0,22 μM, 0,44 μM et 10 μM de composé No. 6 correspondant à 0%, 25%, 50%, 75% et 100% d'efficacité d'inhibition de l'efflux d'après la Figure 3. La représentation en double inverse (panneau de droite) montre que les droites coupent l'axe des x en un point unique, typique d'un type d'inhibition non

compétitive.

**Liste des références**

1. GOTTESMAN M. M., LING V. FEBS Lett., 2006, 580(4), 998-1009

2. Juliano, R. L. & Ling, V. A surface glycoprotein modulating drug permeability in Chinese hamster ovary cell mutants. Biochim Biophys Acta 455, 152-162, doi:0005-2736(76)90160-7 [pii] (1976)

3. Cole, S. P. et al. Overexpression of a transporter gene in a multidrug-resistant human lung cancer cell line. Science 258, 1650-1654 (1992)

4. DOYLE L. A., YANG W., ABRUZZO L. V., KROGMANN T., GAO Y., RISHI A. K., ROSS D. D. Proc. Natl. Acad. Sci. USA, 1998, 95(26), 15665-15670

5. Ross, D. D. et al. Atypical multidrug résistance: breast cancer resistance protein messenger RNA expression in mitoxantrone-selected cell lines. J Natl Cancer Inst 91, 429-433 (1999)

6. J. Cell Sci., 2000, 113(Pt 11), 2011-2021

7. Aller, S. G. et al. Structure of P-Glycoprotein Reveals a Molecular Basis for Poly-Specific Drug Binding. Science 323, 1718-1722, doi:10.1126/science.1168750 (2009)

8. Dawson, R. J. & Locher, K. P. Structure of a bacterial multidrug ABC transporter. Nature 443, 180-185 (2006)

9. Ward, A., Reyes, C. L., Yu, J., Roth, C. B. & Chang, G. Flexibility in the ABC transporter MsbA: Alternating access with a twist. Proceedings of the National Academy of Sciences 104, 19005-19010, doi:10.1073/pnas.0709388104 (2007)

10. Ueda, K., Cardarelli, C., Gottesman, M. M. & Pastan, I. Expression of a Full-Length cDNA for the Human "MDR1" Gene Confers Resistance to Colchicine, Doxorubicin, and Vinblastine. Proceedings of the National Academy of Sciences 84, 3004-3008, doi:10.1073/pnas.84.9.3004 (1987)

11. Litman, T. et al. The multidrug-resistant phenotype associated with overexpression of the new ABC half-transporter, MXR (ABCG2). J Cell Sci 113 (Pt 11), 2011-2021 (2000)

12. Benderra, Z. et al. Breast Cancer Resistance Protein and P-Glycoprotein in 149 Adult Acute Myeloid Leukemias. Clin Cancer Res 10, 7896-7902, doi:10.1158/1078-0432.ccr-04-0795 (2004)

13. Juliano, R. L. & Ling, V. A surface glycoprotein modulating drug permeability in Chinese hamster ovary cell mutants. Biochim Biophys Acta 455, 152-162, doi:0005-2736(76)90160-7 [pii] (1976)

14. W. Clark Still, Michael Kahn, and Abhijit Mitra J. Org. Chem. Rapid Chromatographic Technique for Preparative Separations with Moderate Resolution, J. Org. Chem., 1978, 43(14), 2923-2925

15. Kim, R. B. Drug transporters in HIV Therapy. Top HIV Med 11, 136-139 (2003)

16. ALLIKMENTS R., SCHRIML L. M., HUTCHINSON A., ROMANO-SPICA V., DEAN M. Cancer Res., 1998, 58 (23), 5337-5339

17. COORAY H. C., JANVILISRI T., VAN VEEN H. W., HLADKY S. B., BARRAND M. A. Biochem. Biophys. Res. Commun., 2004, 317(1), 269-275

18. JONKER J. W., SMIT J. W., BRINKHUIS R. F., MALIEPAARD M., BEIJNEN J.H., SCHELLENS J. H. J. Natl. Cancer Inst., 2000, 92, 1651-1656

19. Modok, S., Mellor, H. R. & Callaghan, R. Modulation of multidrug resistance efflux pump activity to overcome chemoresistance in cancer. Current Opinion in Pharmacology 6, 350-354 (2006)

20. Current Opinion in Pharmacology, 2006, 6, 350-354

21. SARKADI B., SEPRŐDI J., CSUKA O., MAGOCSI M., MEZÔ I., PALYI I., TEPLÁN I., VADÁSZ Z., VINCZE, B. US 6,297,216 B1, 2 octobre 2001

22. SEPRŐDI J., MEZÔ I., VADÁSZ Zs., SZABÔ K., SARKADI B., TEPLÁN I. in peptides 1996, Proceedings of the Twenty-Fourth European Peptide Symposium ; September 8-13, 1996, Edinburgh, Scotland. Robert RAMAGE and Roger EPTON (Eds), 1998, pp 801-802

23. SHAROM F. J., YU X., LU P., LIU R., CHU J. W. K., SZABÓ K., MULLER M., HOSE C. D., MONKS A., VARADI A., SEPRŐDI J., SARKADI B. Biochem. Pharmacol., 1999, 58(4), 571-586

24. Thèse, Synthèse et étude biologique d'analogues di- et tripeptidiques de réversines susceptibles de moduler l'activité de deux protéines de transport, la glycorpotéine P et BCRP (2007)

25. Lai, M. Y. H. et al. Synthesis and pharmacological evaluation of glycine-modified analogues of the neuroprotective agent glycyl-l-prolyl-l-glutamic acid (GPE). Bioorganic & Medicinal Chemistry 13, 533-548 (2005)

26. Martinez, J. et al. Synthesis and biological activities of some pseudo-peptide analogs of tetragastrin: the importance of the peptide backbone. Journal of Medicinal Chemistry 28, 1874-1879 (1985)

27. Palyi, I. et al. Compounds for reversing drug resistance. Hungary patent (2001)

28. SEKO T., KATO M., KOHNO H., ONO S., HASHIMURA K., TAKIMIZU H., NAKAI K., MAEGAWA H., KATSUBE N., TODA M. Bioorg. Med. Chem., 2003, 11(8), 1901-1913

29. FEHRENTZ J.-A., CASTRO B. Synthesis, 1983, 676-678

30. MARTINEZ J., BALI J . P., RODRIGUEZ M., CASTRO B., MAGOUS R., LAUR J., LIGNON M-F. J. Med. Chem., 1985, 28(12), 1874-1879

31. CARRASCO M. R., BROWN R. T. J. Org. Chem., 2003, 68(23), 8853-8858

32. Robey R.W. et al. 2003, Br. J. Cancer 89(10) 1971-1978

33. Cardarelli et al. 1995 Cancer Res 55 1086-1091

34. Vogel's textbook of practical organic chemistry, Ve édition, 1989, Longman Scientific & Technical éd.

## Revendications

**1.** Utilisation d'un composé de formule (I) ou d'un de ses sels :

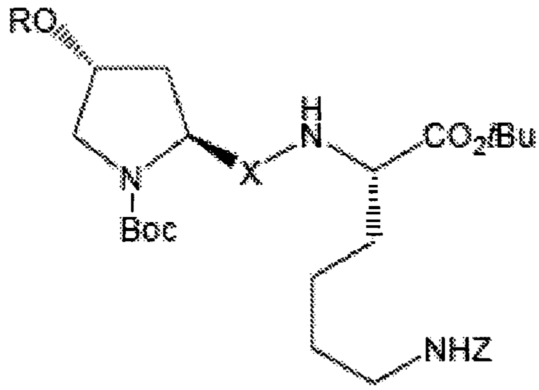

(I)

pour la préparation d'un médicament, dans laquelle
R représente -Bn, ou -COBn, ou -COcHex, ou $-CH_2cHex$,
X représente $-CH_2$ ou -CO,
Z représente un groupe benzyloxycarbonyle
les abréviations Bn et cHex étant utilisées respectivement pour benzyle et cyclohexyle.

2. Utilisation selon la revendication 1, dans laquelle ledit médicament est destiné au traitement d'un cancer ou d'une infection.

3. Utilisation selon l'une quelconque des revendications 1 ou 2, dans laquelle ledit médicament est un adjuvant au traitement chimiothérapeutique d'un cancer ou d'une infection.

4. Utilisation selon l'une quelconque des revendications précédentes, dans laquelle ledit composé de structure (I) a une concentration de demi inhibition maximale ($IC_{50}$) d'environ 0,22 $\mu$M.

5. Utilisation selon l'une quelconque des revendications précédentes, dans laquelle ledit médicament est administré avec un agent chimiothérapeutique.

6. Utilisation selon la revendication 5, dans laquelle ledit agent chimiothérapeutique est un agent anti-cancéreux ou un agent anti-infectieux.

7. Utilisation selon l'une quelconque des revendications précédentes, dans laquelle ledit médicament est destiné à réduire l'activité de la protéine d'efflux ABCB1 chez un mammifère.

8. Utilisation selon l'une quelconque des revendications précédentes, dans laquelle ledit médicament est destiné à diminuer la résistance à ladite substance active du point de vue pharmaceutique.

9. Utilisation selon l'une quelconque des revendications précédentes, dans laquelle :

   R représente -Bn et X représente $CH_2$, ou
   R représente -COBn et X représente CO, ou
   R représente -COcHex et X représente CO, ou
   R représente -Bn et X représente CO, ou
   R représente $-CH_2cHex$ et X représente CO.

10. Composé de structure (I) ou un de ses sels tel que défini dans la revendication 1, pour son utilisation comme médicament.

11. Composé de structure (I) ou un de ses sels tel que défini dans la revendication 1, pour son utilisation dans le traitement d'un cancer ou d'une infection.

12. Composition pharmaceutique **caractérisée en ce qu'**elle comprend un composé de structure (I) ou un de ses sels

et un support pharmaceutiquement acceptable.

**13.** Composition pharmaceutique selon la revendication 12, dans laquelle la concentration dudit composé de structure (I) est comprise entre 0,001 à 500 $\mu$M.

**14.** Composition selon l'une quelconque des revendications 12 à 13, comprenant en outre un agent chimiothérapeutique.

**15.** Utilisation d'un composé de structure (I) pour inhiber *in vitro* la pompe d'efflux ABCB1.

**Patentansprüche**

**1.** Verwendung einer Verbindung der Formel (I) oder eines ihrer Salze :

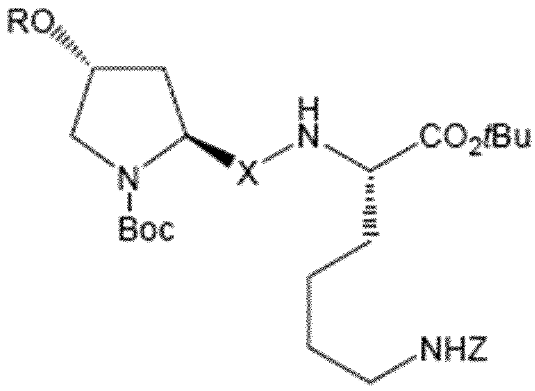

(I)

zur Herstellung eines Medikaments, in welchem
R für -Bn, oder -COBn, oder -COcHex, oder -$CH_2$cHex steht,
X für -$CH_2$ oder -CO steht,
Z für eine Benzyloxycarbonylgruppe steht,
wobei die Abkürzungen Bn und cHex jeweils für Benzyl und Cyclohexyl verwendet werden.

**2.** Verwendung nach Anspruch 1, wobei das Medikament zur Behandlung eines Krebses oder einer Infektion bestimmt ist.

**3.** Verwendung nach Anspruch 1 oder 2, wobei das Medikament ein Adjuvans für die chemotherapeutische Behandlung eines Krebses oder einer Infektion ist.

**4.** Verwendung nach einem der vorstehenden Ansprüche, wobei die Verbindung der Struktur (I) eine maximale halbe Inhibitionskonzentration ($IC_{50}$) von ungefähr 0,22 $\mu$M aufweist.

**5.** Verwendung nach einem der vorstehenden Ansprüche, wobei das Medikament mit einem chemotherapeuthischen Mittel verabreicht wird.

**6.** Verwendung nach Anspruch 5, wobei das chemotherapeuthische Mittel ein Mittel gegen Krebs oder ein Anti-Infektionsmittel ist.

**7.** Verwendung nach einem der vorstehenden Ansprüche, wobei das Medikament zur Verringerung der Aktivität des Effluxproteins ABCB1 bei einem Säugetier bestimmt ist.

**8.** Verwendung nach einem der vorstehenden Ansprüche, wobei das Medikament dazu bestimmt ist, die Resistenz gegen die aktive Substanz unter pharmazeutischen Gesichtspunkten zu verringern.

**9.** Verwendung nach einem der vorstehenden Ansprüche, wobei :

R für -Bn und X für $CH_2$ steht, oder
R für -COBn und X für CO steht, oder
R für -COcHex und X für CO steht, oder
R für -Bn und X für CO steht, oder
R für $-CH_2$cHex und X für CO steht.

**10.** Verbindung der Struktur (I) oder ein Salz hiervon gemäß der Definition in Anspruch 1 zur Verwendung als Medikament.

**11.** Verbindung der Struktur (I) oder ein Salz hiervon gemäß der Definition in Anspruch 1 zur Behandlung eines Krebses oder einer Infektion.

**12.** Pharmazeutische Verbindung, **dadurch gekennzeichnet, dass** sie eine Verbindung der Struktur (I) oder ein Salz hiervon und einen pharmazeutisch akzeptablen Träger umfasst.

**13.** Pharmazeutische Verbindung nach Anspruch 12, in welcher die Konzentration der Verbindung der Struktur (I) zwischen 0,001 und 500 μM beträgt.

**14.** Verbindung nach einem der Ansprüche 12 bis 13, ferner ein chemotherapeutisches Mittel umfassend.

**15.** Verwendung einer Verbindung der Struktur (I), um *in vitro* die Effluxpumpe ABCB1 zu inhibieren.

## Claims

**1.** Use of a compound of formula (I) or a salt thereof:

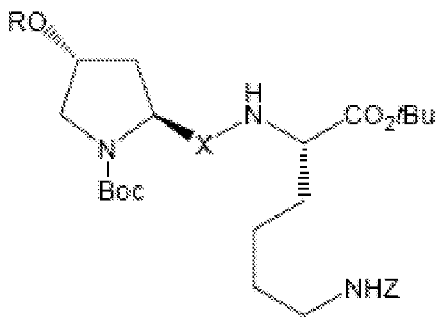

(I)

for preparing a medicament, wherein:

R represents -Bn, -COBn, -COcHex or $-CH_2$cHex,
X represents $-CH_2$ or -CO,
Z represents a benzyloxycarbonyl group,
Bn and cHex abbreviations being respectively used for benzyl and cyclohexyl

**2.** Use according to claim 1, wherein said medicament is intended for treating a cancer or an infection.

**3.** Use according to any one of claims 1 or 2, wherein said medicament is an adjuvant to a chemotherapy treatment of a cancer or of an infection.

**4.** Use according to any one of the preceding claims, wherein said compound of structure (I) has a half-maximum inhibition concentration ($IC_{50}$) of about 0.22 μM.

**5.** Use according to any one of the preceding claims, wherein said medicament is administered with a chemotherapeutic agent.

**6.** Use according to claim 5, wherein said chemotherapeutic agent is an anticancer agent or an anti-infective agent.

**7.** Use according to any one of the preceding claims, wherein said medicament is intended for reducing the activity of the ABCB1 efflux protein in a mammal.

**8.** Use according to any one of the preceding claims, wherein said medicament is intended for reducing the resistance to said pharmaceutically active substance.

**9.** Use according to any one of the preceding claims, wherein:

R represents -Bn and X represents $CH_2$, or
R represents -COBn and X represents CO, or
R represents -CocHex and X represents CO, or
R represents -Bn and X represents CO, or
R represents -$CH_2$cHex and X represents CO.

**10.** Compound of structure (I) or a salt thereof, as defined in claim 1, for use thereof as a medicament.

**11.** Compound of structure (I) or a salt thereof, as defined in claim 1, for use thereof in the treatment of a cancer or of an infection.

**12.** Pharmaceutical composition **characterized in that** it comprises a compound of structure (I) or a salt thereof and a pharmaceutically acceptable carrier.

**13.** Pharmaceutical composition according to claim 12, wherein the concentration of said compound of structure (I) is between 0.001 and 500 μM.

**14.** Composition according to any one of claims 12 to 13, further comprising a chemotherapeutic agent.

**15.** Use of a compound of structure (I) for inhibiting the ABCB1 efflux pump *in vitro.*

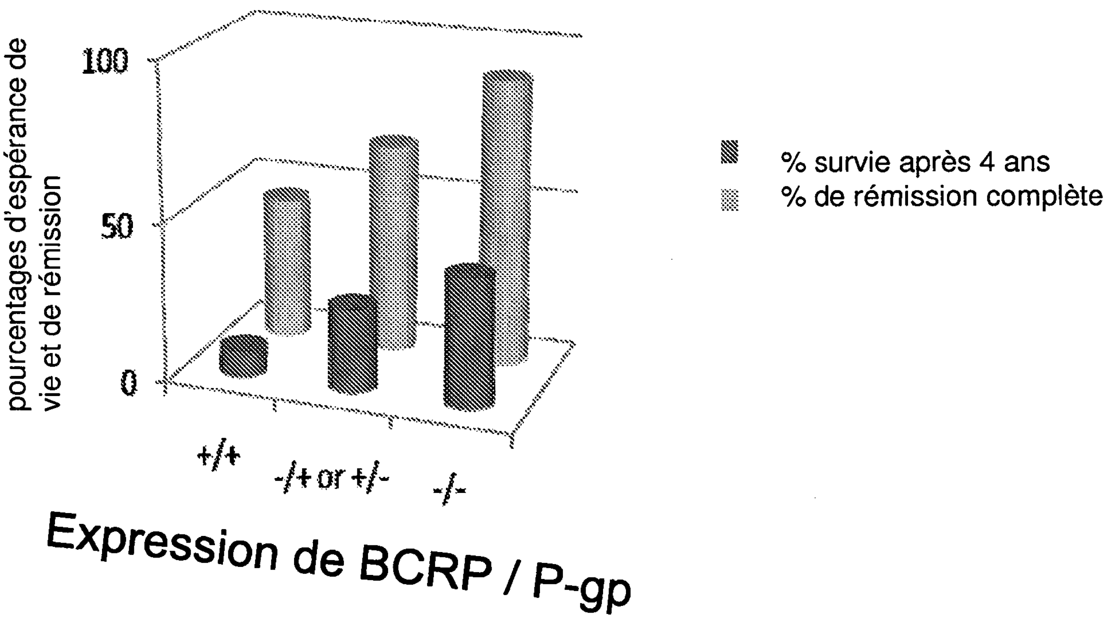
pourcentages d'espérance de vie et de rémission
100
50
0
+/+
-/+ or +/-
-/-
Expression de BCRP / P-gp
% survie après 4 ans
% de rémission complète

Fig. 1

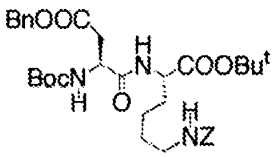

**Réversine 121**
**#1**. 75.8±1.6%
$IC_{50}$: 1.41±0.34 µM

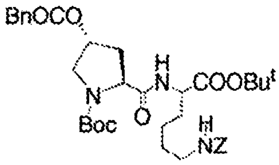

**#2**. 68.2±10.2%

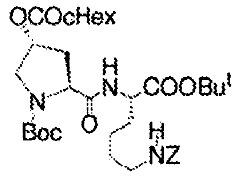

**#3**. 105.7±13.4%
$IC_{50}$: 1.68±0.28 M

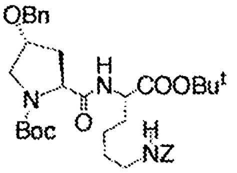

**#4**. 99.7±11.0%
$IC_{50}$: 0.74±0.22 µM

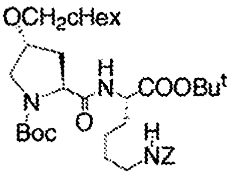

**#5**. 82.2±10.8%
$IC_{50}$: 1.13±0.34 µM

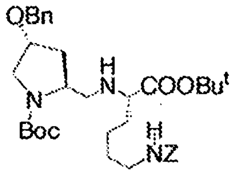

**#6**. 106.9±23%
IC50 : 0.22±0.03 µM

Fig. 2

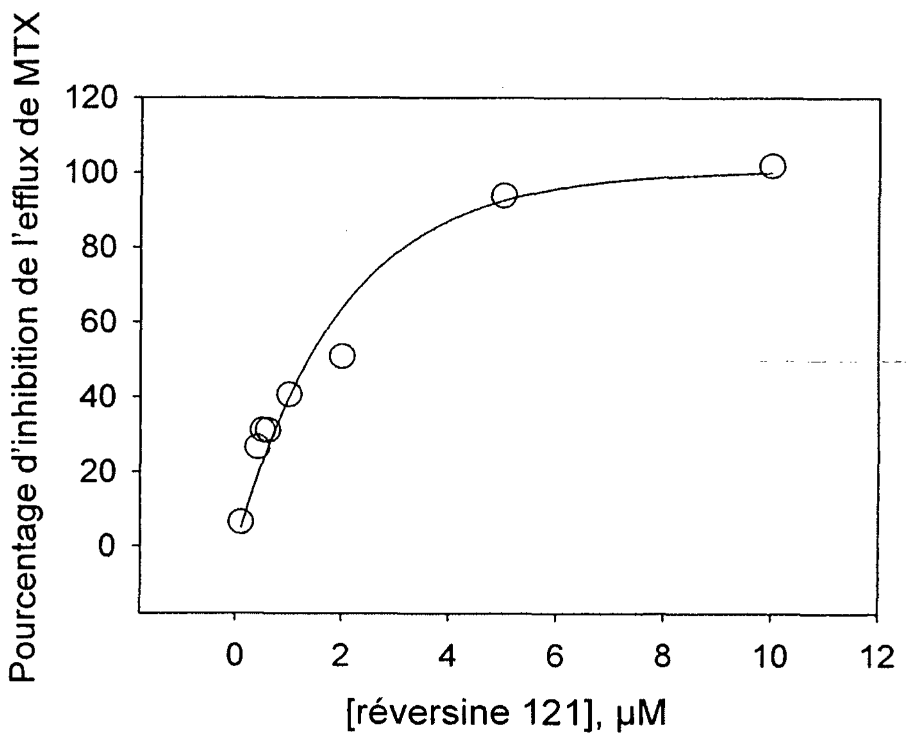
Pourcentage d'inhibition de l'efflux de MTX
120
100
80
60
40
20
0
0
2
4
6
8
10
12
[réversine 121], µM

Fig. 3 A

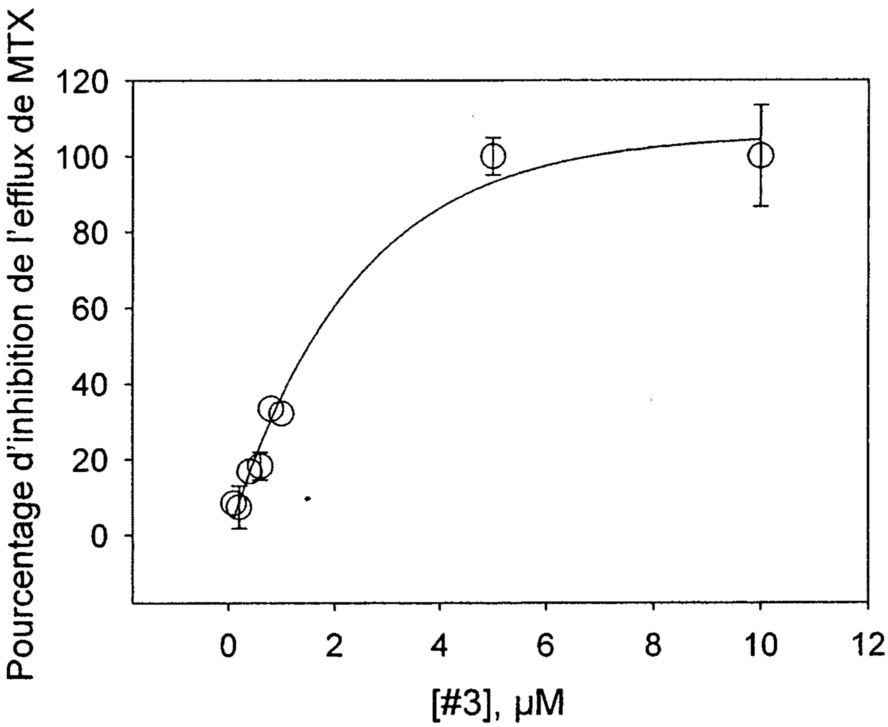
Pourcentage d'inhibition de l'efflux de MTX
120
100
80
60
40
20
0
0
2
4
6
8
10
12
[#3], µM

Fig. 3 B

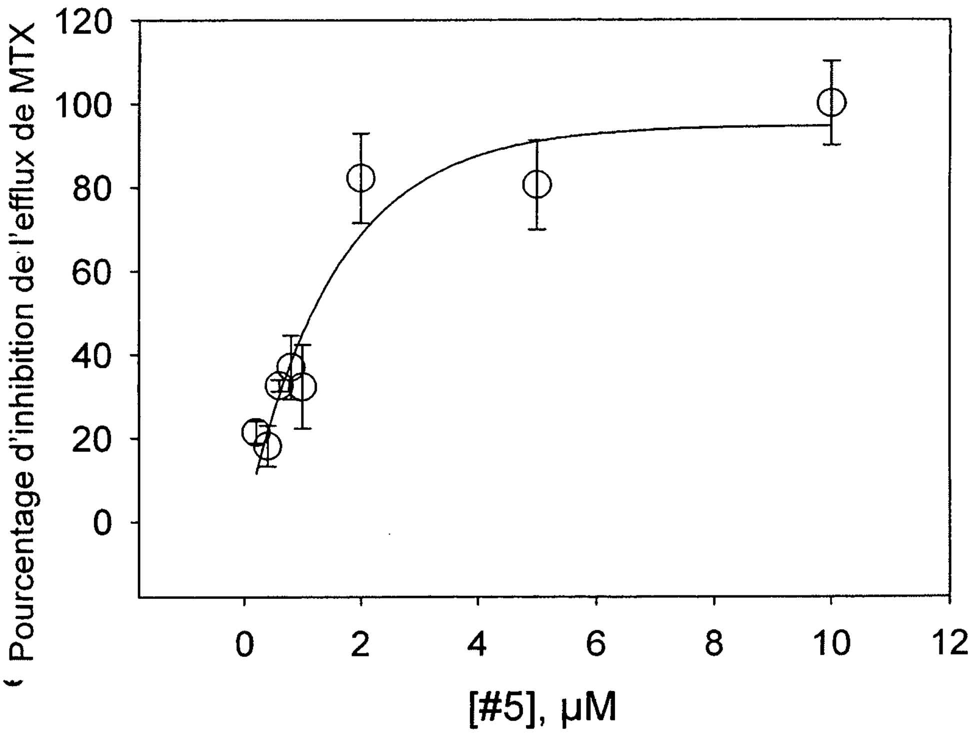
Pourcentage d'inhibition de l'efflux de MTX
120
100
80
60
40
20
0
0
2
4
6
8
10
12
[#5], µM

Fig. 3 C

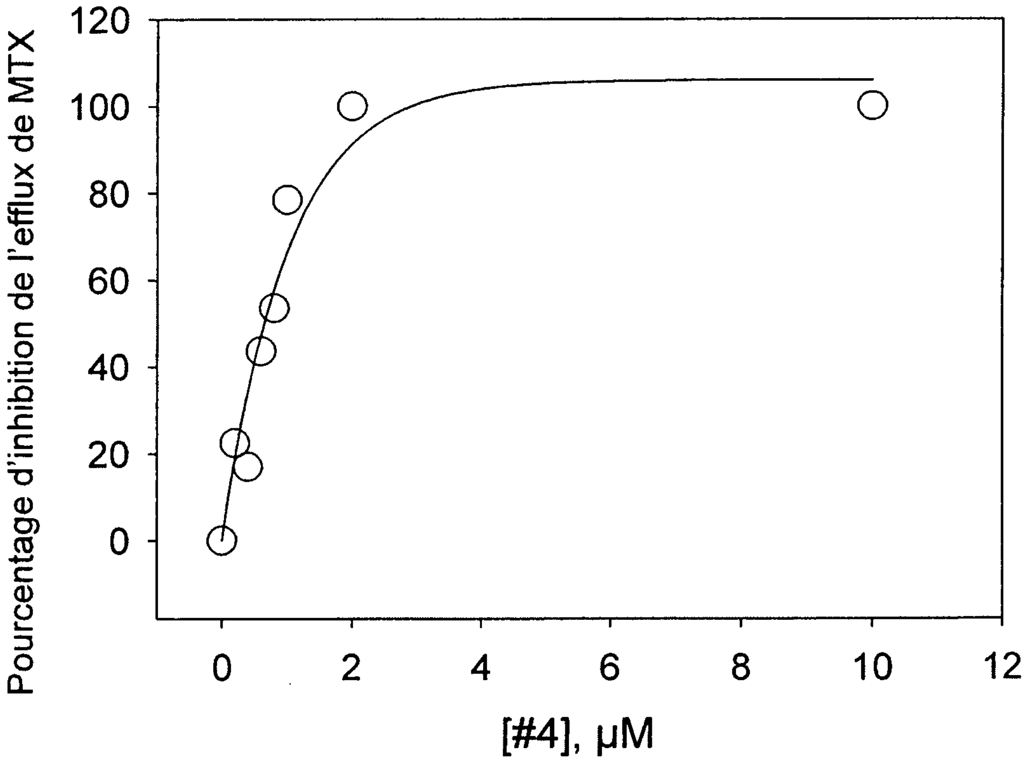
Pourcentage d'inhibition de l'efflux de MTX
120
100
80
60
40
20
0
0
2
4
6
8
10
12
[#4], µM

Fig. 3 D

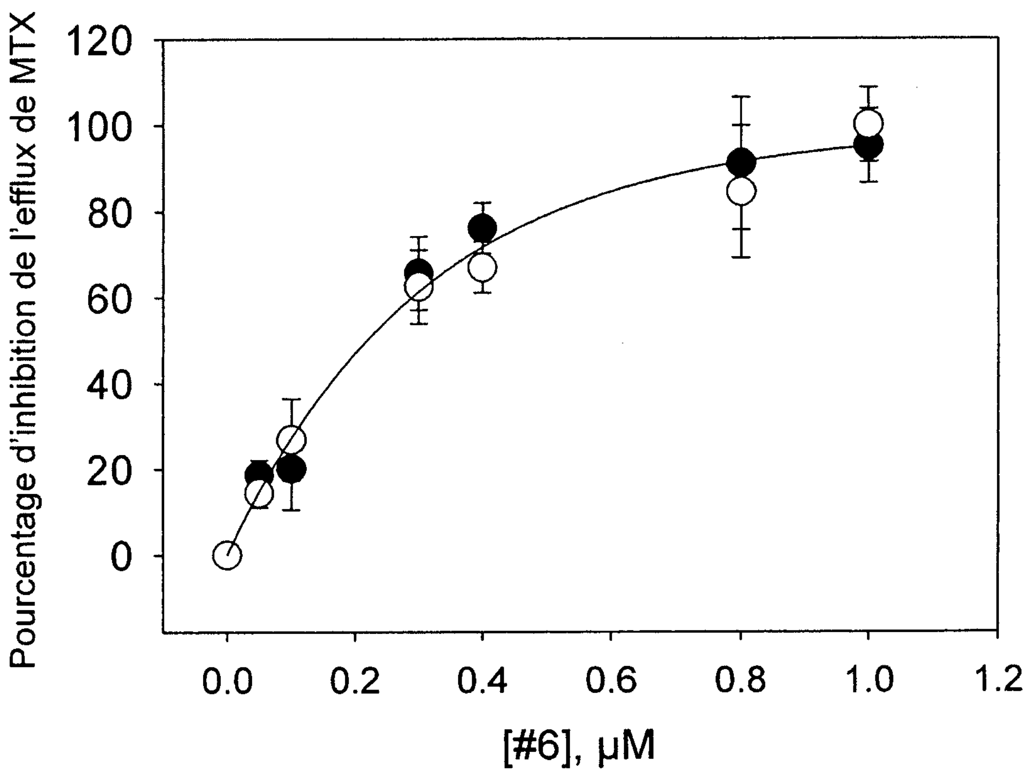
Pourcentage d'inhibition de l'efflux de MTX
120
100
80
60
40
20
0
0.0
0.2
0.4
0.6
0.8
1.0
1.2
[#6], µM

Fig. 3 E

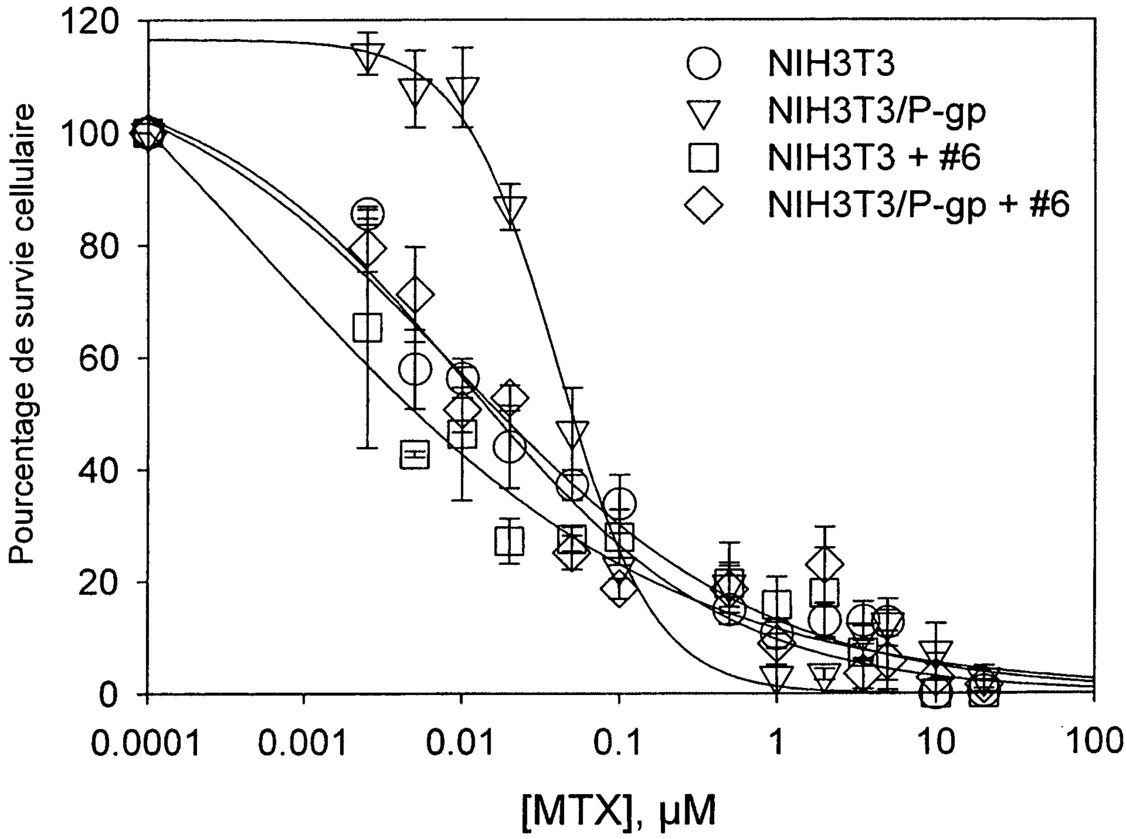
120
100
80
60
40
20
0
Pourcentage de survie cellulaire
NIH3T3
NIH3T3/P-gp
NIH3T3 + #6
NIH3T3/P-gp + #6
0.0001
0.001
0.01
0.1
1
10
100
[MTX], µM

Fig. 4

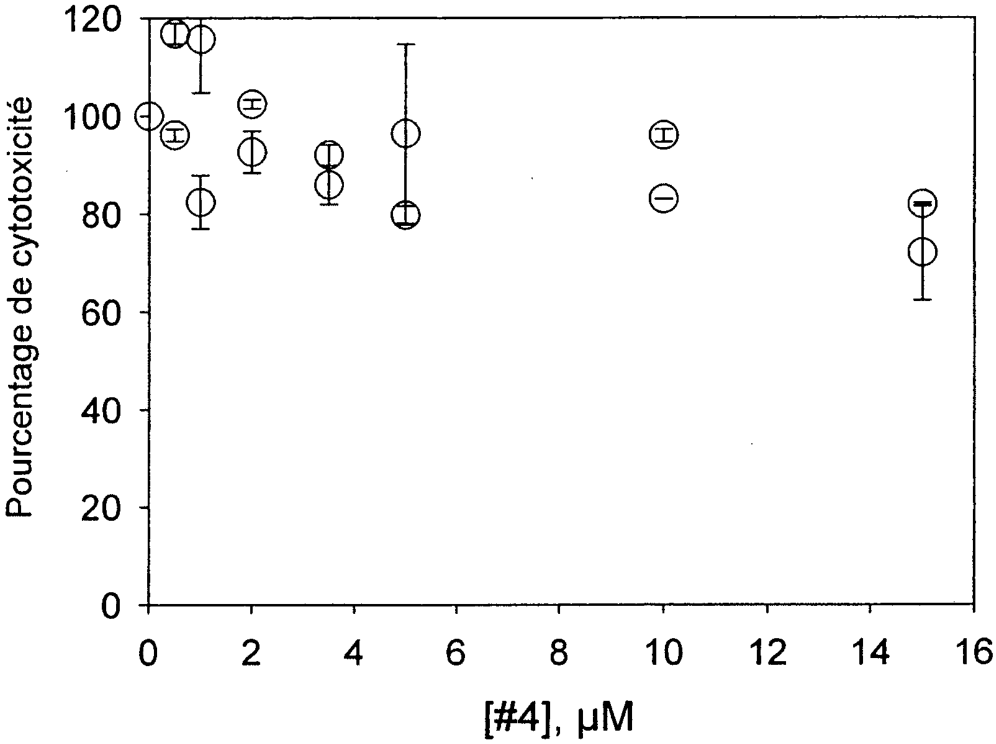
120
100
80
60
40
20
0
Pourcentage de cytotoxicité
0
2
4
6
8
10
12
14
16
[#4], µM

Fig. 5 A

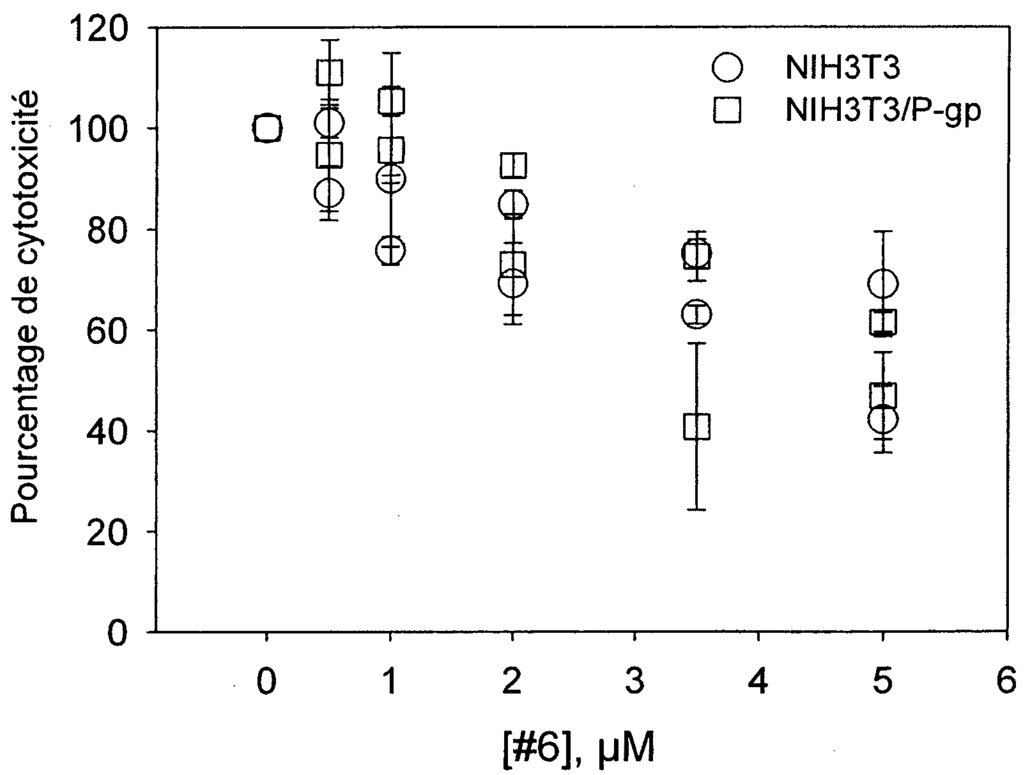
120
100
80
60
40
20
0
Pourcentage de cytotoxicité
NIH3T3
NIH3T3/P-gp
0
1
2
3
4
5
6
[#6], µM

Fig. 5 B

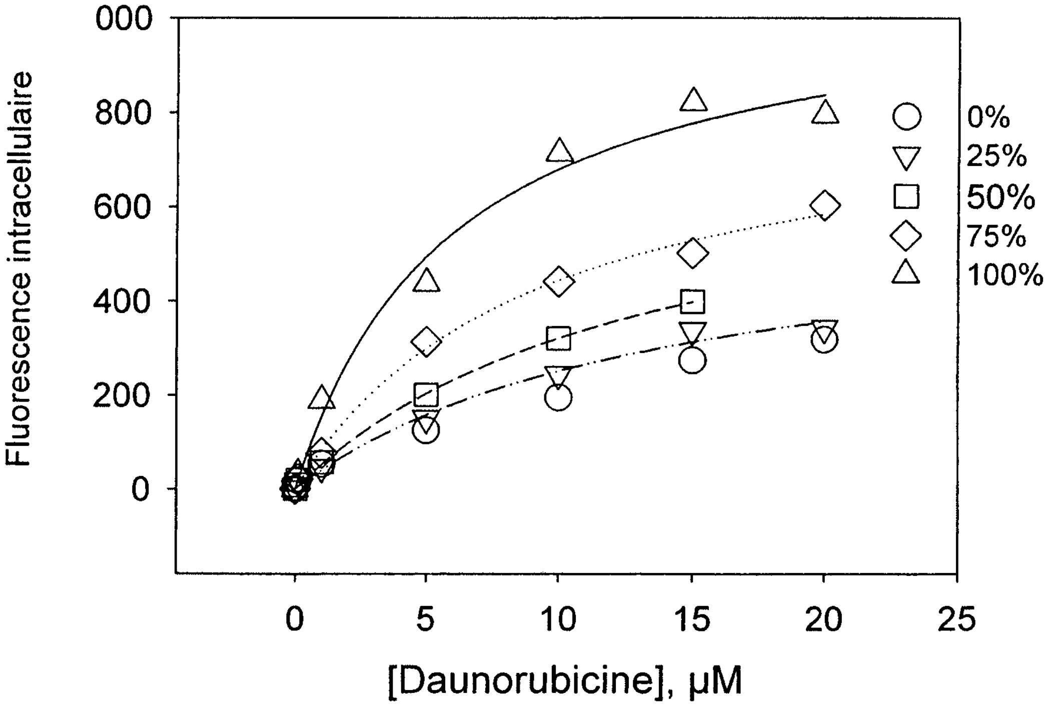
000
800
600
400
200
0
Fluorescence intracellulaire
0
5
10
15
20
25
[Daunorubicine], µM
0%
25%
50%
75%
100%

Fig. 6 A

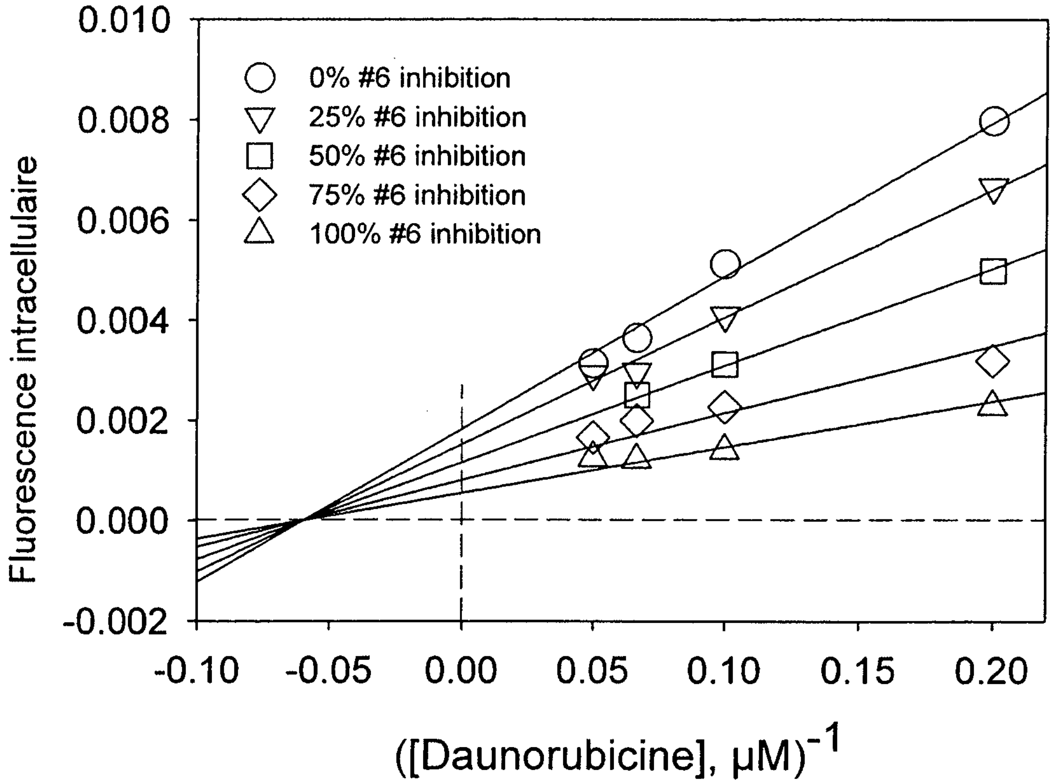
0.010
0.008
0.006
0.004
0.002
0.000
-0.002
Fluorescence intracellulaire
0% #6 inhibition
25% #6 inhibition
50% #6 inhibition
75% #6 inhibition
100% #6 inhibition
-0.10
-0.05
0.00
0.05
0.10
0.15
0.20
([Daunorubicine], µM)-1

Fig. 6 B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6297216 B1, SARKADI B., SEPRŐDI J., CSUKA O., MAGOCSI M., MEZÔ I., PALYI I., TEPLÁN I., VADÁSZ Z., VINCZE, B.
- US 6297216 B


### Littérature non-brevet citée dans la description


- **GOTTESMAN M. M. ; LING V.** *FEBS Lett.,* 2006, vol. 580 (4), 998-1009
- **JULIANO, R. L. ; LING, V.** A surface glycoprotein modulating drug permeability in Chinese hamster ovary cell mutants. *Biochim Biophys Acta,* 1997, vol. 455, 152-162
- **COLE, S. P. et al.** Overexpression of a transporter gene in a multidrug-resistant human lung cancer cell line. *Science,* 1992, vol. 258, 1650-1654
- **DOYLE L. A. ; YANG W. ; ABRUZZO L. V. ; KROGMANN T. ; GAO Y. ; RISHI A. K. ; ROSS D. D.** *Proc. Natl. Acad. Sci. USA,* 1998, vol. 95 (26), 15665-15670
- **ROSS, D. D. et al.** Atypical multidrug résistance: breast cancer resistance protein messenger RNA expression in mitoxantrone-selected cell lines. *J Natl Cancer Inst,* 1999, vol. 91, 429-433
- **BATES et al.** *J. Cell Sci.,* 2000, vol. 113, 2011-2021
- **ALLER, S. G. et al.** Structure of P-Glycoprotein Reveals a Molecular Basis for Poly-Specific Drug Binding. *Science,* 2009, vol. 323, 1718-1722
- **DAWSON, R. J. ; LOCHER, K. P.** Structure of a bacterial multidrug ABC transporter. *Nature,* 2006, vol. 443, 180-185
- **WARD, A. ; REYES, C. L. ; YU, J. ; ROTH, C. B. ; CHANG, G.** Flexibility in the ABC transporter MsbA: Alternating access with a twist. *Proceedings of the National Academy of Sciences,* 2007, vol. 104, 19005-19010
- **UEDA, K. ; CARDARELLI, C. ; GOTTESMAN, M. M. ; PASTAN, I.** Expression of a Full-Length cDNA for the Human "MDR1" Gene Confers Resistance to Colchicine, Doxorubicin, and Vinblastine. *Proceedings of the National Academy of Sciences,* 1987, vol. 84, 3004-3008
- **LITMAN, T. et al.** The multidrug-resistant phenotype associated with overexpression of the new ABC half-transporter, MXR (ABCG2). *J Cell Sci,* 2000, vol. 113, 2011-2021
- **JULIANO, R. L. ; LING, V.** A surface glycoprotein modulating drug permeability in Chinese hamster ovary cell mutants. *Biochim Biophys Acta,* 1976, vol. 455, 152-162
- **KIM, R. B.** Drug transporters in HIV Therapy. *Top HIV Med,* 2003, vol. 11, 136-139
- **ALLIKMENTS R. ; SCHRIML L. M. ; HUTCHINSON A. ; ROMANO-SPICA V. ; DEAN M.** *Cancer Res.,* 1998, vol. 58, 5337-5339
- **COORAY H. C. ; JANVILISRI T. ; VAN VEEN H. W. ; HLADKY S. B. ; BARRAND M. A.** *Biochem. Biophys. Res. Commun.,* 2004, vol. 317 (1), 269-275
- **JONKER J. W. ; SMIT J. W. ; BRINKHUIS R. F. ; MALIEPAARD M. ; BEIJNEN J.H. ; SCHELLENS J. H.** *J. Natl. Cancer Inst,* 2000, vol. 92, 1651-1656
- **BENDERRA, Z. et al.** Breast Cancer Resistance Protein and P-glycoprotein in 149 Adult Acute Myeloid Leukemias. *Clin Cancer Res,* 2004, vol. 10, 7896-7902
- **MODOK, S. ; MELLOR, H. R. ; CALLAGHAN, R.** Modulation of multidrug resistance efflux pump activity to overcome chemoresistance in cancer. *Current Opinion in Pharmacology,* 2006, vol. 6, 350-354
- peptides 1996. **SEPRŐDI J ; MEZÔ I. ; VADÁSZ ZS. ; SZABÓ K. ; SARKADI B. ; TEPLÁN I.** Proceedings of the Twenty-Fourth European Peptide Symposium. 08 Septembre 1996, 801-802
- **SHAROM F. J. ; YU X. ; LU P. ; LIU R. ; CHU J. W. K. ; SZABÔ K. ; MULLER M. ; HOSE C. D. ; MONKS A. ; VARADI A.** *Biochem. Pharmacol.,* 1999, vol. 58 (4), 571-586
- **LAI, M. Y. H. et al.** Synthesis and pharmacological evaluation of glycine-modified analogues of the neuroprotective agent glycyl-l-prolyl-l-glutamic acid (GPE. *Bioorganic & Medicinal Chemistry,* 2005, vol. 13, 533-548

- **MARTINEZ, J. et al.** Synthesis and biological activities of some pseudo-peptide analogs of tetragastrin: the importance of the peptide backbone. *Journal of Medicinal Chemistry,* 1985, vol. 28, 1874-1879
- **W. CLARK STILL ; MICHAEL KAHN ; ABHIJIT MITRA.** J. Org. Chem. Rapid Chromatographic Technique for Preparative Separations with Moderate Resolution. *J. Org. Chem,* 1978, vol. 43 (14), 2923-2925
- Vogel's textbook of practical organic chemistry. Longman Scientific & Technical, 1989
- **PALYI, I. et al.** Compounds for reversing drug resistance. *Hungary,* 2001
- **CARRASCO M. R. ; BROWN R. T.** *J. Org. Chem.,* 2003, vol. 68 (23), 8853-8858
- **SEKO T. ; KATO M. ; KOHNO H. ; ONO S. ; HASHIMURA K. ; TAKIMIZU H. ; NAKAI K. ; MAEGAWA H. ; KATSUBE N. ; TODA M.** *Bioorg. Med. Chem.,* 2003, vol. 11 (8), 1901-1913
- **FEHRENTZ J.-A. ; CASTRO B.** *Synthesis,* 1983, 676-678
- **MARTINEZ J. ; BALI J . P. ; RODRIGUEZ M. ; CASTRO B. ; MAGOUS R. ; LAUR J. ; LIGNON M-F.** *J. Med. Chem.,* 1985, vol. 28 (12), 1874-1879
- **ROBEY R.W. et al.** *Br. J. Cancer,* 2003, vol. 89 (10), 1971-1978
- **CARDARELLI et al.** *Cancer Res,* 1995, vol. 55, 1086-1091
- *J. Cell Sci.,* 2000, vol. 113, 2011-2021
- **DAWSON, R. J. ; LOCHER, K. P.** Structure of a bacterial multidrug ABC transporter. *Nature,* 2006, vol. 443, 180-185
- **WARD, A. ; REYES, C. L. ; YU, J. ; ROTH, C. B. ; CHANG, G.** Flexibility in the ABC transporter MsbA: Alternating access with a twist. *Proceedings of the National Academy of Sciences,* 2007, vol. 104, 19005-19010
- **LITMAN, T. et al.** The multidrug-resistant phenotype associated with overexpression of the new ABC half-transporter, MXR (ABCG2. *J Cell Sci,* 2000, vol. 113, 2011-2021
- **BENDERRA, Z. et al.** Breast Cancer Resistance Protein and P-Glycoprotein in 149 Adult Acute Myeloid Leukemias. *Clin Cancer Res,* 2004, vol. 10, 7896-7902
- **W. CLARK STILL ; MICHAEL KAHN ; ABHIJIT MITRA.** J. Org. Chem. Rapid Chromatographic Technique for Preparative Separations with Moderate Resolution. *J. Org. Chem.,* 1978, vol. 43 (14), 2923-2925
- **ALLIKMENTS R. ; SCHRIML L. M. ; HUTCHINSON A. ; ROMANO-SPICA V. ; DEAN M.** *Cancer Res.,* 1998, vol. 58 (23), 5337-5339
- **JONKER J. W. ; SMIT J. W. ; BRINKHUIS R. F. ; MALIEPAARD M. ; BEIJNEN J.H. ; SCHELLENS J. H.** *J. Natl. Cancer Inst.,* 2000, vol. 92, 1651-1656
- *Current Opinion in Pharmacology,* 2006, vol. 6, 350-354
- peptides 1996. **DI J. ; MEZÔ I. ; VADÁSZ ZS. ; SZABÔ K. ; SARKADI B. ; TEPLÁN I.** Proceedings of the Twenty-Fourth European Peptide Symposium. 08 Septembre 1996, 801-802
- **SHAROM F. J. ; YU X. ; LU P. ; LIU R. ; CHU J. W. K. ; SZABÓ K. ; MULLER M. ; HOSE C. D. ; MONKS A. ; VARADI A.** *Biochem. Pharmacol.,* 1999, vol. 58 (4), 571-586
- Thèse, Synthèse et étude biologique d'analogues di- et tripeptidiques de réversines susceptibles de moduler l'activité de deux protéines de transport. *la glycorpotéine P et BCRP,* 2007
- **PALYI, I. et al.** *Compounds for reversing drug resistance,* 2001